# EUROPÄISCHE PATENTSCHRIFT

(11) **EP 1 515 931 B1**
(45) Veröffentlichungstag und Bekanntmachung des Hinweises auf die Patenterteilung: **22.11.2006**
(21) Anmeldenummer: 03760649.8
(22) Anmeldetag: 18.06.2003
(51) Int. Cl.: C07C 23/18, C07D 309/04, G02F 1/13, C09K 19/30, C09K 19/34

(54) **3,3,4,4-TETRAFLUORCYCLOPENTANVERBINDUNGEN ALS KOMPONENTEN FLÜSSIGKRISTALLINER MEDIEN**
3,3,4,4-TETRAFLUOROCYCLOPENTANE COMPOUNDS, USED AS COMPONENTS OF LIQUID CRYSTAL MEDIA
COMPOSES DE 3,3,4,4-TETRAFLUOROCYCLOPENTANE EN TANT QUE COMPOSANTES DE MILIEUX DE CRISTAUX LIQUIDES

(30) Priorität: 24.06.2002 DE 10228183
(43) Veröffentlichungstag der Anmeldung: 23.03.2005
(73) Patentinhaber: Merck Patent GmbH, 64293 Darmstadt (DE)
(72) Erfinder: REIFFENRATH, Volker, 64380 Rossdorf (DE); LÜSSEM, Georg, 85238 Peterhausen (DE)
(86) Internationale Anmeldenummer: PCT/EP2003/006447
(87) Internationale Veröffentlichungsnummer: WO 2004/000771

(56) Entgegenhaltungen:
- EP-A- 0 789 067
- DE-A- 19 607 999
- DE-A- 19 918 009
- DE-A- 19 941 654

## Beschreibung

Die Erfindung betrifft 3,3,4,4-Tetrafluorcyclopentanverbindungen der Formel I, 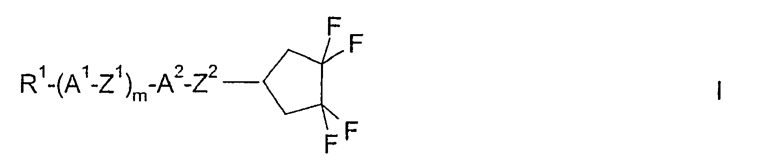 worin
- R¹: H, einen unsubstituierten, einen einfach durch CN oder CF₃ oder einen mindestens einfach durch Halogen substituierten Alkylrest mit bis zu 15 C-Atomen, wobei in diesen Resten auch eine oder mehrere CH₂-Gruppen jeweils unabhängig voneinander durch -O-, -CH=CH-, -S-, 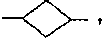 -CO-, -CO-O-, -O-CO- oder -O-CO-O- so ersetzt sein können, dass O-Atome nicht direkt miteinander verknüpft sind,
- A¹ und A²: jeweils unabhängig voneinander

(a) trans-1,4-Cyclohexylenrest, worin auch eine oder mehrere nicht benachbarte CH₂-Gruppen durch -O- und/oder -S-ersetzt sein können,
(b) 1,4-Phenylenrest, worin auch eine oder zwei CH-Gruppen durch N ersetzt sein können,
(c) 1,4-Cyclohexenylen,
(d) Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) bis (d) durch ein oder mehrere, insbesondere ein oder zwei, Fluoratome substituiert sein können,
- Z₁ und Z₂: jeweils unabhängig voneinander -CO-O-, -O-CO-, -CH₂O-, -OCH₂-, -CH₂CH₂-, -CH=CH-, -C=C-, -(CH₂)₄-, -CF₂O-, -OCF₂-, -C₂F₄-, -CH=CH-CH₂CH₂- oder eine Einfachbindung,
- m: 0, 1 oder 2,
bedeuten.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Im Hinblick auf die verschiedensten Einsatzbereiche derartiger Verbindungen mit hohem Δε war es jedoch wünschenswert, weitere Verbindungen mit hoher Nematogenität zur Verfügung zu haben, die auf die jeweiligen Anwendungen genau maßgeschneiderte Eigenschaften aufweisen.

Es wurde nun gefunden, dass Verbindungen der Formel I als Komponenten flüssigkristalliner Medien vorzüglich geeignet sind. Insbesondere verfügen sie über vergleichsweise niedrige Viskositäten. Mit den erfindungsgemäßen Verbindungen ist es möglich Mischungen mit einem sehr niedrigen Δn, hohem positiven Δε und sehr hoher Voltage Holding Ratio herzustellen. Derartige Mischungen sind insbesondere für reflektive Anwendungen geeignet. Mit ihrer Hilfe lassen sich stabile flüssigkristalline Medien mit breitem Mesophasenbereich und vorteilhaften Werten für die optische Anisotropie (Δn) und dielektrische Anisotropie (Δε) erhalten. Die erfindungsgemäßen Medien weisen ferner ein sehr gutes Tieftemperaturverhalten auf.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Der Einfachheit halber bedeuten im folgenden A³ einen Rest der Formel 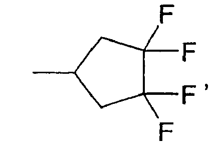 Cyc einen 1,4-Cyclohexylrest, Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest, wobei Cyc und/oder Phe unsubstituiert oder ein- oder zweifach durch F substituiert sein können.

A¹ und A² sind vorzugsweise ausgewählt aus der Gruppe Cyc, Che, Phe, Pyr, Pyd und Dio.

Die Verbindungen der Formel I umfassen dementsprechend Verbindungen mit zwei Ringen der Teilformeln Ia und Ib:

R¹-A²-A³ Ia

R¹-A²-Z²-A³ Ib

Verbindungen mit drei Ringen der Teilformeln Ic bis le:

R¹-A¹-A²-A³ Ic

R¹-A¹-Z¹-A²-A³ Id

R¹-A¹-A²-Z²-A³ Ie

sowie Verbindungen mit vier Ringen der Teilformeln If bis Ii:

R¹-A¹-A¹-A²-A³ If

R¹-A¹-Z¹-A¹-A²-A³ Ig

R¹-A¹-A¹-A²-Z²-A³ Ih

R¹-A¹-Z¹-A¹-A²-Z²-A³ Ii

Bevorzugt sind auch Verbindungen der Formel I sowie aller Teilformeln, in denen A¹ und/oder A² ein- oder zweifach durch F substituiertes 1,4-Phenylen bedeutet. Insbesondere sind dies 2-Fluor-1,4-phenylen, 3-Fluor-1,4-phenylen und 3,5-Difluor-1,4-phenylen.

A¹ und A² bedeuten vorzugsweise 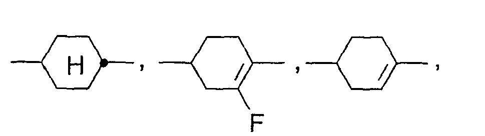 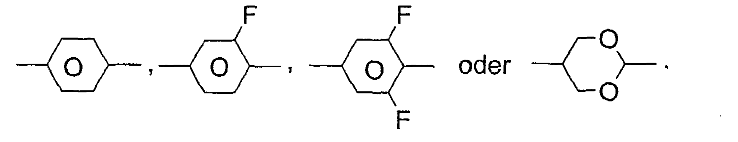 Z¹ und Z² bedeuten bevorzugt eine Einfachbindung, -CO-O-, -O-CO-, -CF₂O- und -CH₂CH₂-, in zweiter Linie bevorzugt -OCF₂-, -CH₂O- und -OCH₂-. Falls einer der Reste Z¹ und Z² -(CH₂)₄- oder -CH=CH-CH₂CH₂₋bedeutet, so ist der andere Rest Z¹ oder Z² (falls vorhanden) vorzugsweise eine Einfachbindung.

R¹ bedeutet vorzugsweise Alkyl, Alkoxy, Alkenyl oder Alkenyloxy mit bis zu 7 C-Atomen.

m ist vorzugsweise 0 oder 1.

Falls R¹ einen Alkylrest und/oder einen Alkoxyrest bedeutet, so kann dieser geradkettig oder verzweigt sein. Vorzugsweise ist er geradkettig, hat 1, 2, 3, 5, 6 oder 7 C-Atome und bedeutet demnach bevorzugt Methyl, Ethyl, Propyl, Pentyl, Heptyl, Butyl, Hexyl, Ethoxy, Propoxy, Butoxy, Pentoxy,

Hexoxy oder Heptoxy, ferner Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3-oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R¹ einen Alkylrest bedeutet, in dem eine CH₂-Gruppe durch -CH=CH-ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R¹ einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R¹ einen einfach durch CN oder CF₃ substituierten Alkyl- oder Alkenylrest bedeutet, so ist dieser Rest vorzugsweise geradkettig und die Substitution durch CN oder CF₃ in ω̅-Position.

Falls R¹ einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I, die über für Polymerisationsreaktionen geeignete Flügelgruppen R¹ verfügen, eignen sich zur Darstellung flüssigkristalliner Polymerer.

Verbindungen der Formel I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2 Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R¹ einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxyethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxy-carbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Verbindungen der Formel I, die über für Polykondensationen geeignete Flügelgruppen R¹ verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind.

Diejenigen der vorstehend genannten Formeln, die eine oder mehrere Gruppen Pyd, Pyr und/oder Dio enthalten, umschließen jeweils die beiden 2,5-Stellungsisomeren.

Bevorzugte kleinere Gruppen von Verbindungen sind diejenigen der Teilformeln 11 bis 130:

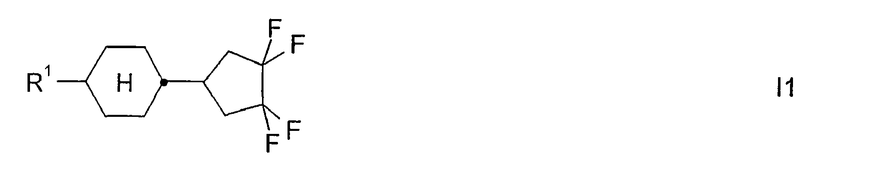

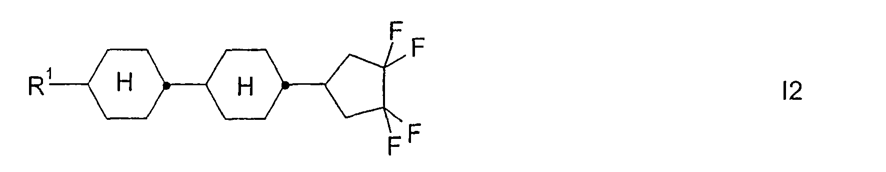

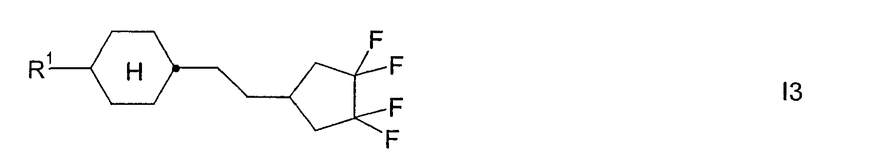

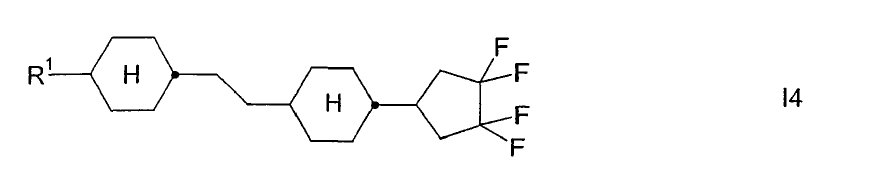

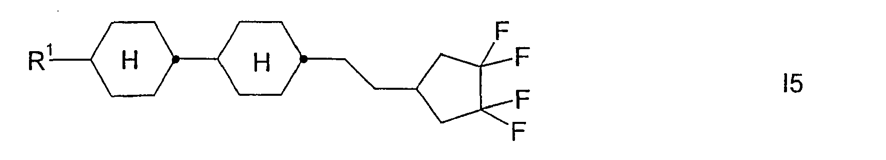

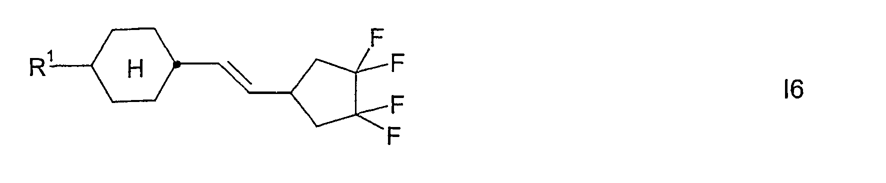

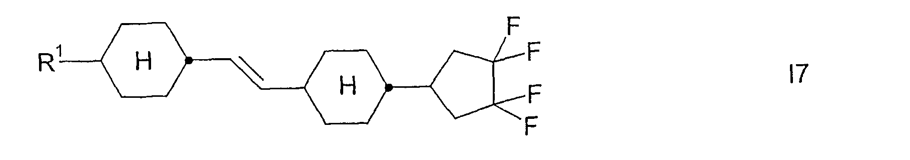

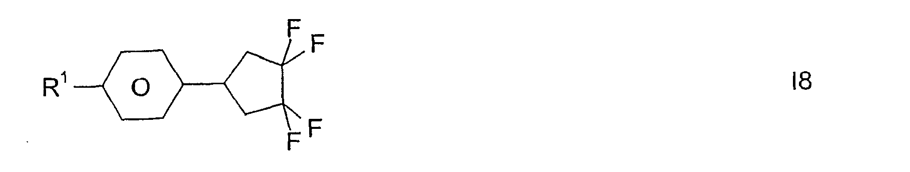

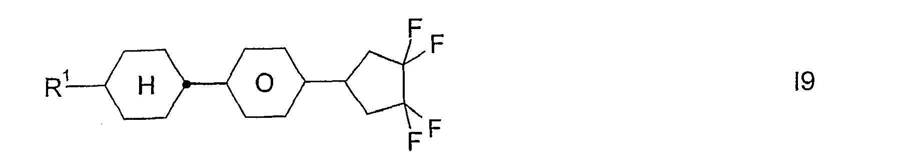

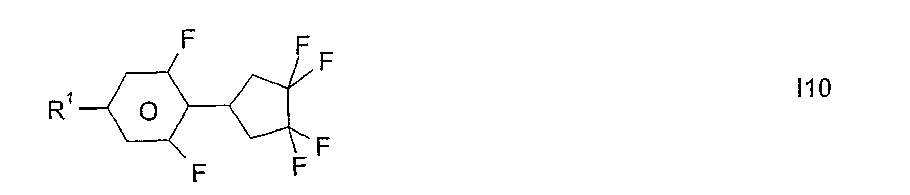

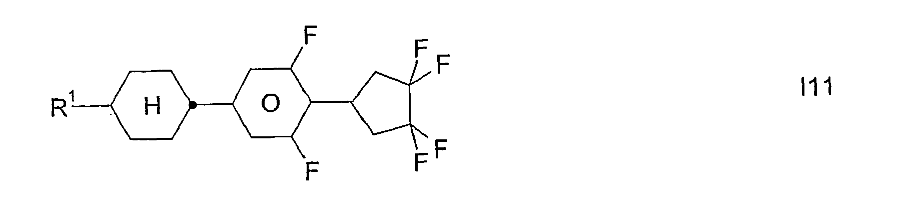

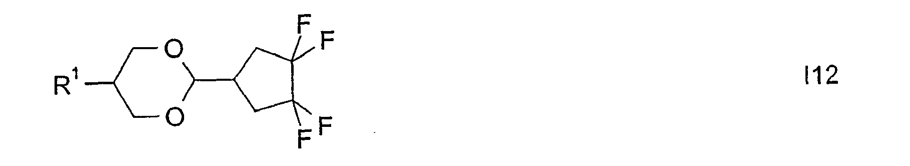

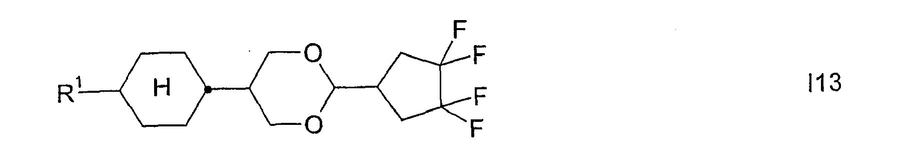

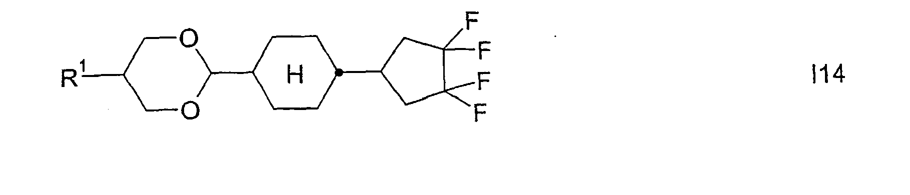

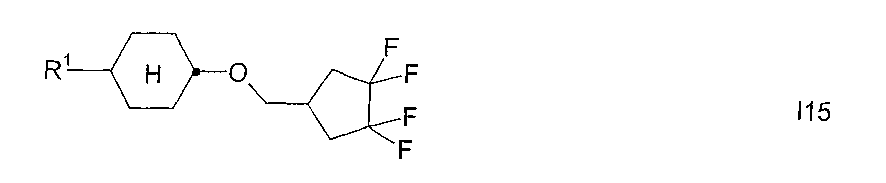

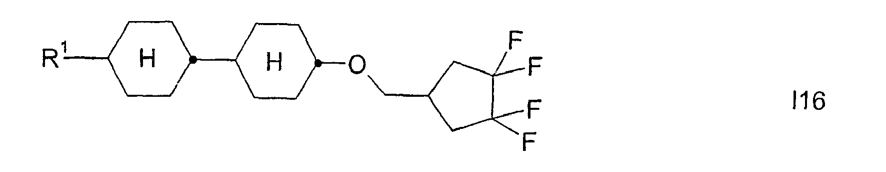

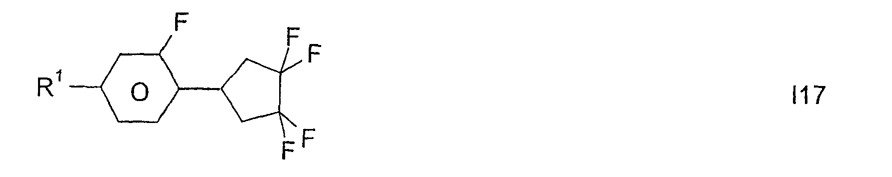

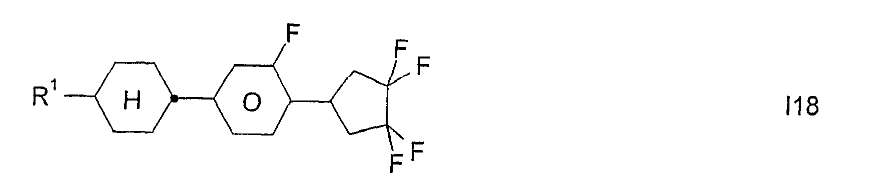

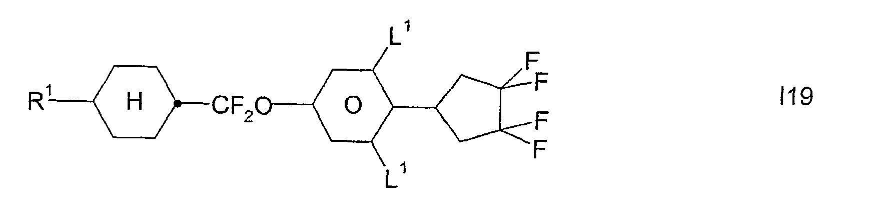

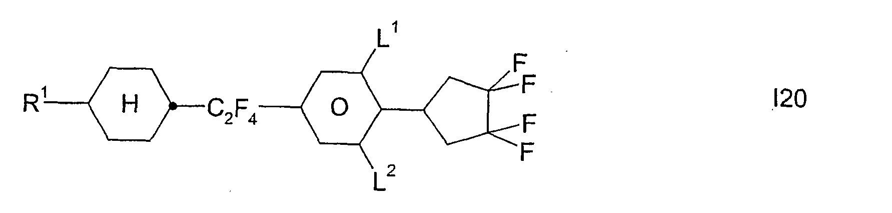

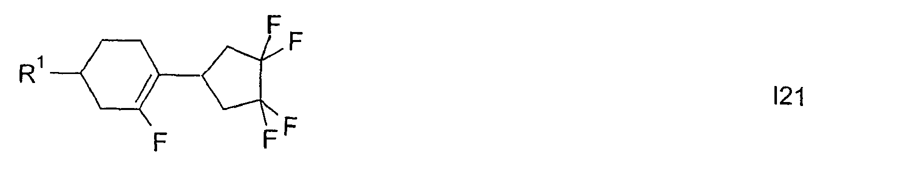

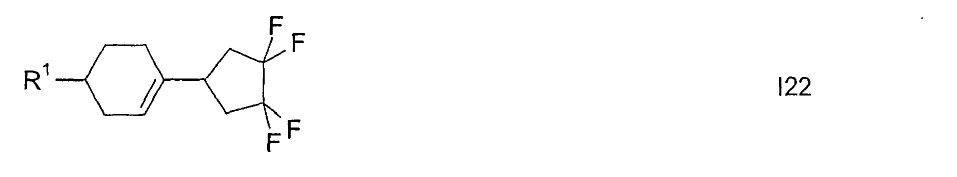

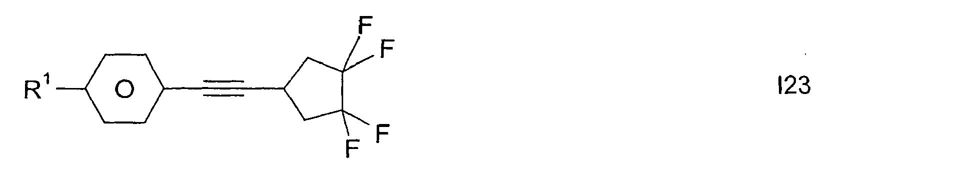

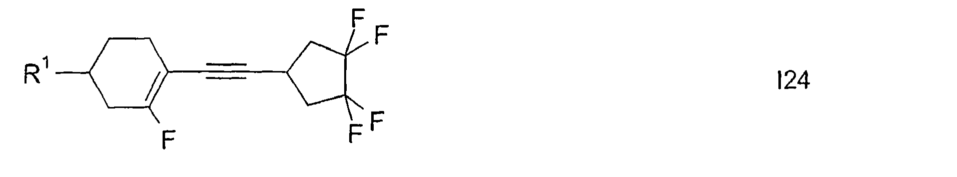

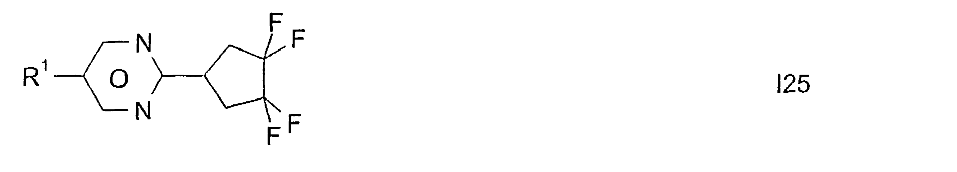

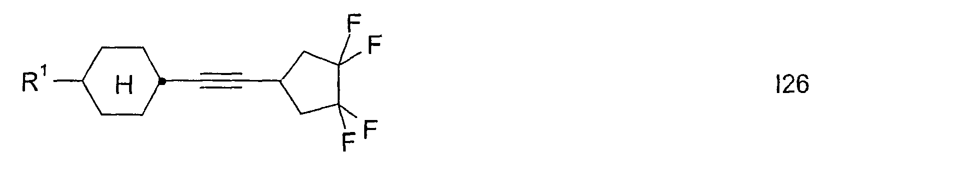

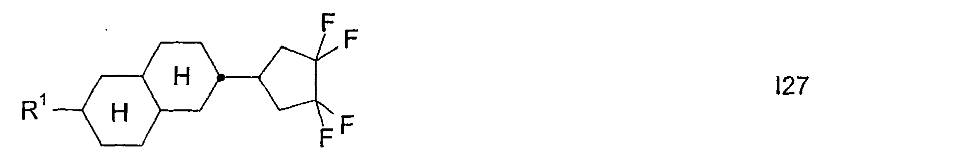

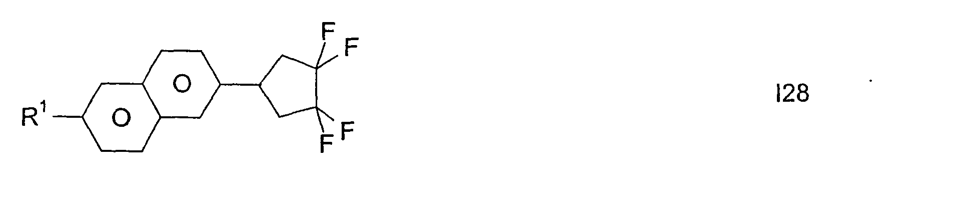

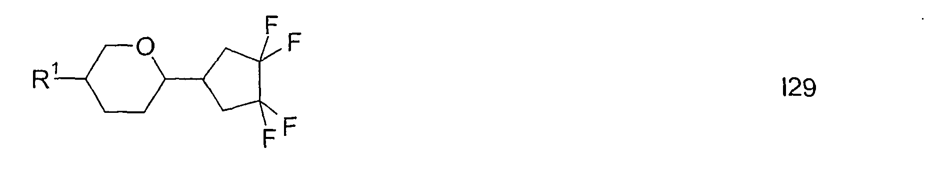

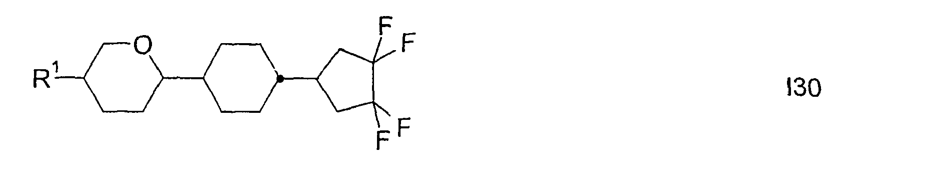

Die 1,4-Cyclohexenylen-Gruppe hat vorzugsweise folgende Strukturen:

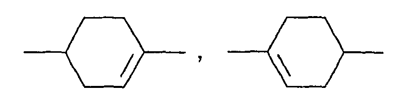

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

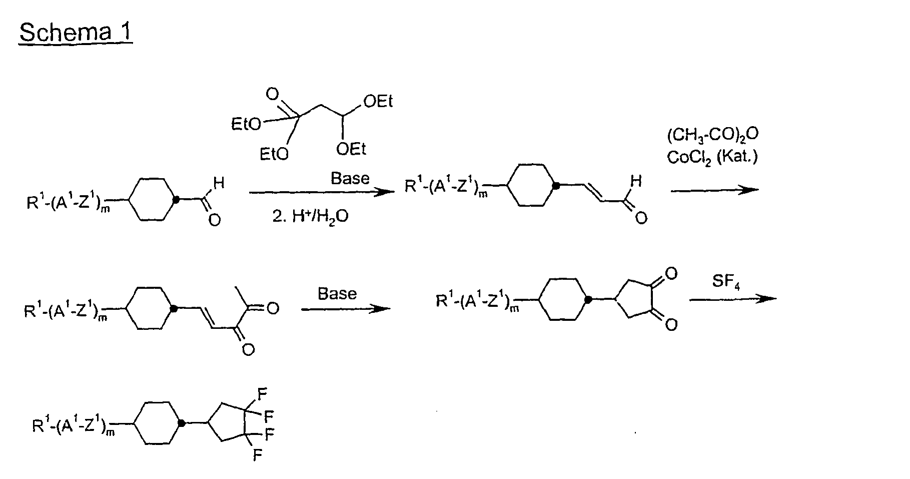

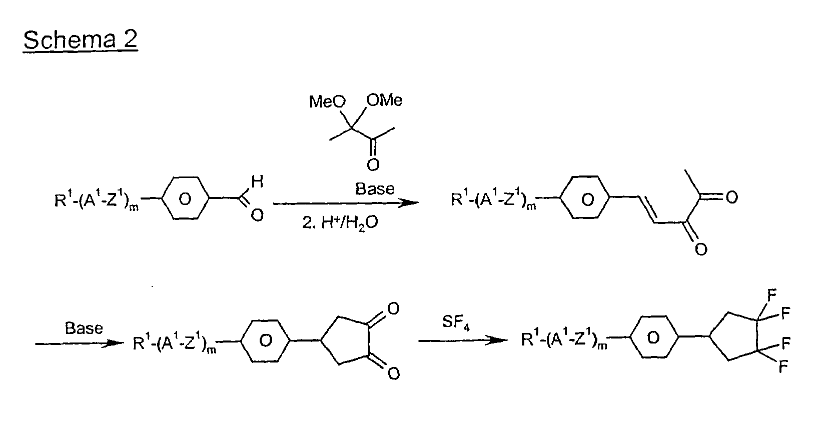

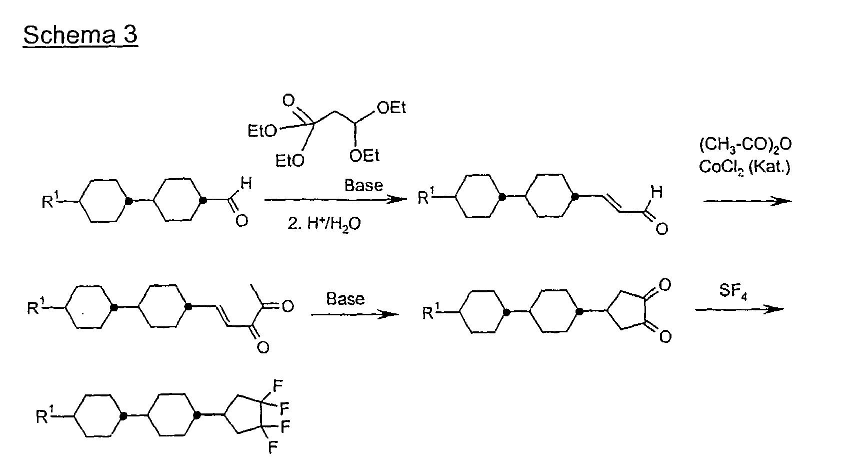

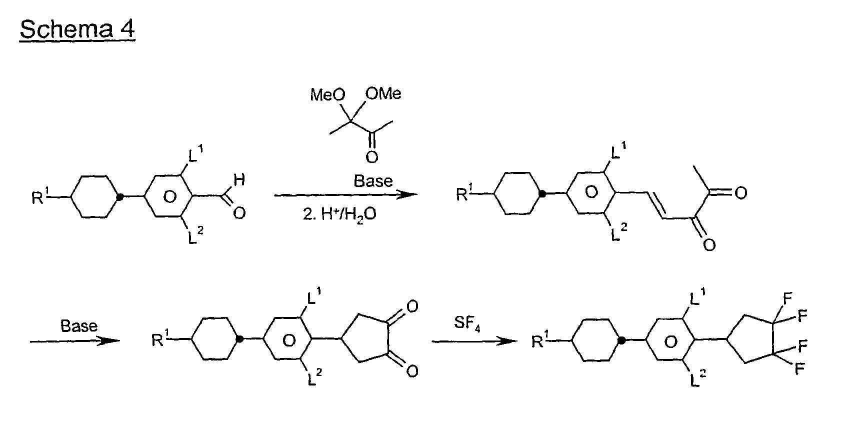

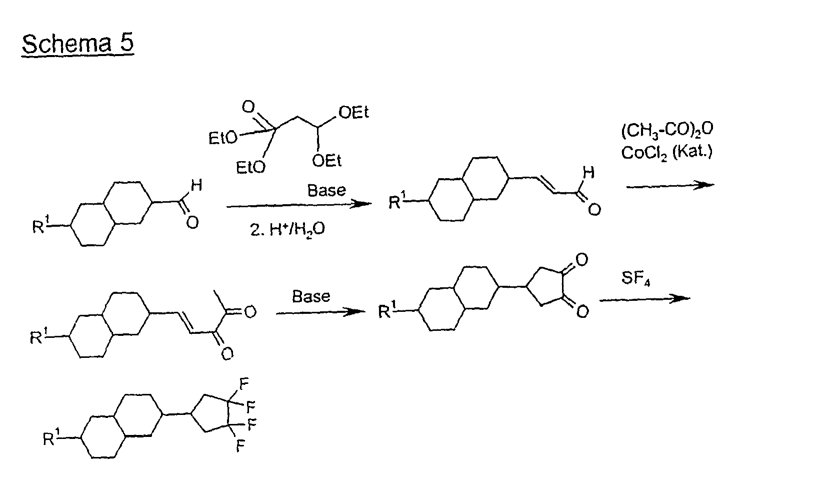

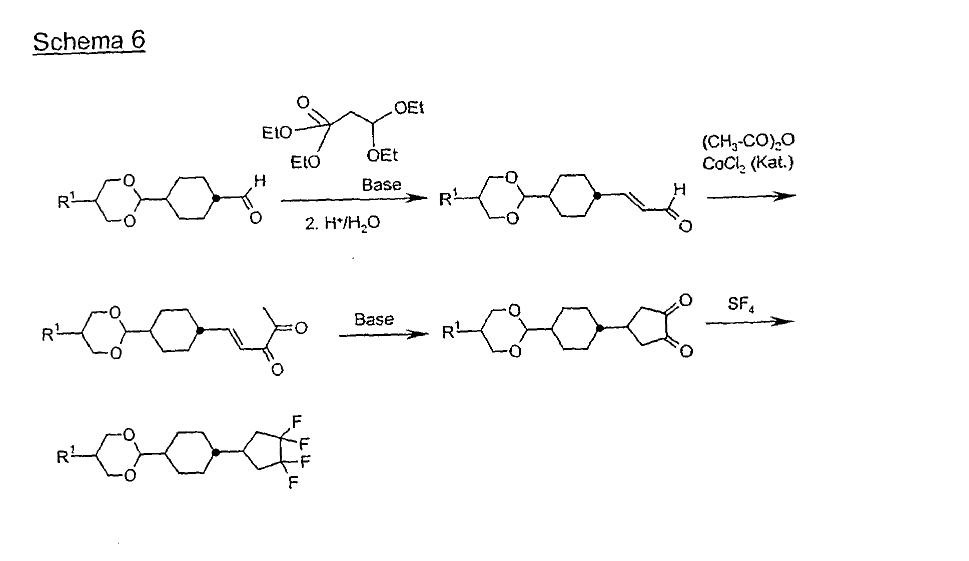

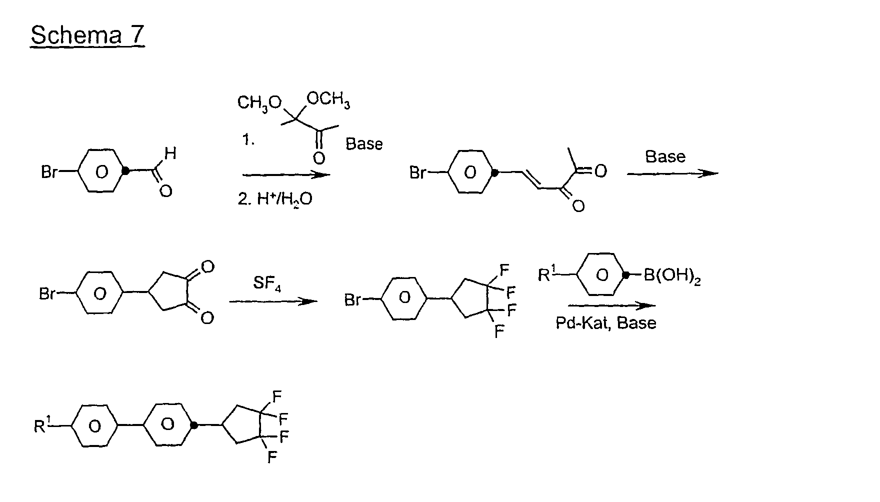

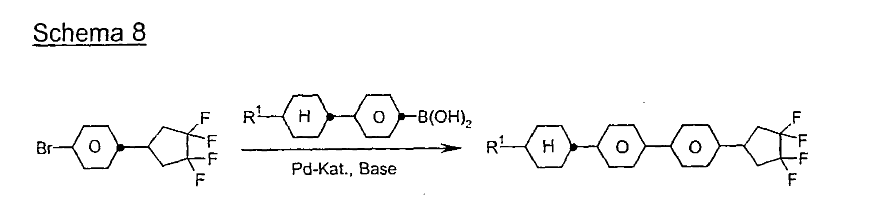

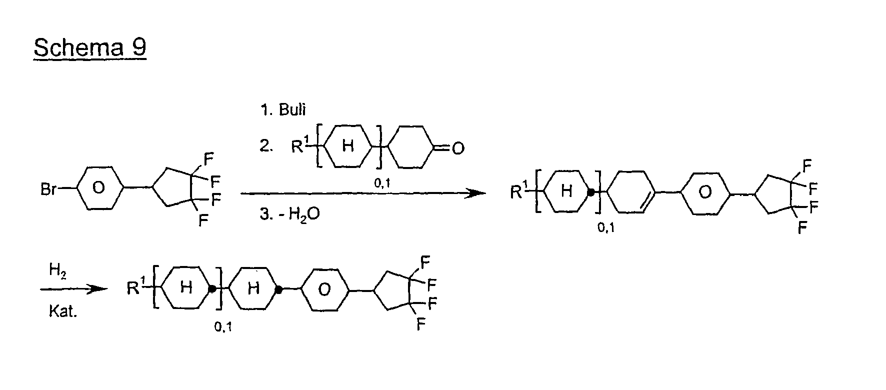

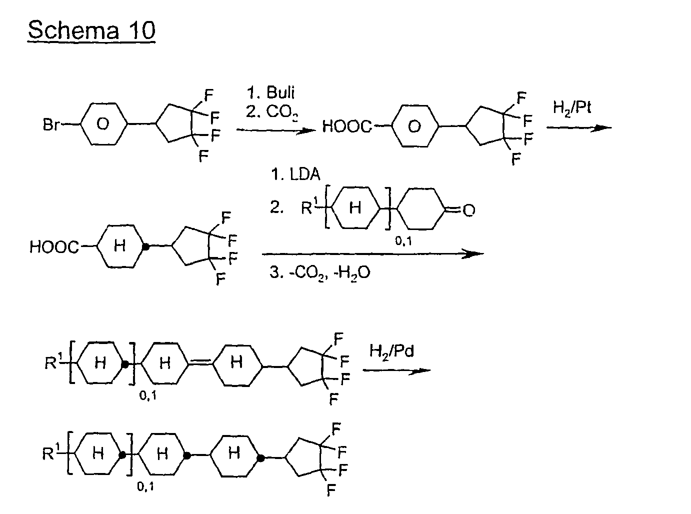

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexyl-ester, Phenyl- oder Cyclohexyl-ester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyr Pyrimidin-2,5-diyl, Pyd Pyridin-2,5-diyl oder Py einen Pyranring bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe, Py und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1 a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢ CH₃₋₍ₖ₊ₗ₎ FₖClₗ, wobei i 0 oder 1 und k+I 1, 2 oder 3 sind; die Verbindungen, in denen R" diese Bedeutung hat, werden mit den Teilformeln 1 b, 2b, 3b, 4b und 5b bezeichnet. Besonders bevorzugt sind solche Verbindungen der Teilformeln 1 b, 2b, 3b, 4b und 5b, in denen R" die Bedeutung -F, -Cl, -NCS, -CF₃, -OCHF₂ oder -OCF₃ hat.

In den Verbindungen der Teilformeln 1 b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1 c, 2c, 3c, 4c und 5c beschrieben. In den Verbindungen der Teilformeln 1c, 2c, 3c, 4c und 5c hat R' die bei den Verbindungen der Teilformeln 1 a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise
- Gruppe A:: 0 bis 90 %, vorzugsweise 20 bis 90 %, insbesondere 30 bis 90 %
- Gruppe B:: 0 bis 80 %, vorzugsweise 10 bis 80 %, insbesondere 10 bis 65 %
- Gruppe C:: 0 bis 80 %, vorzugsweise 5 bis 80 %, insbesondere 5 bis 50 %
wobei die Summe der Massenanteile der in den jeweiligen erfindungsgemäßen Medien enthaltenen Verbindungen aus den Gruppen A und/oder B und/oder C vorzugsweise 5 bis 90 % und insbesondere 10 bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Weiterhin können Stabilisatoren sowie Antioxidantien zugesetzt werden.

Die erfindungsgemäßen Mischungen sind für TN-, STN-, ECB-, IPS-Anwendungen, insbesondere für TFT-Anwendungen, geeignet.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nF.F.F | CₙH₂ₙ₊₁ | F | F | F |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nAm | CₙH₂ₙ₊₁ | COOCₘH₂ₘ₊₁ | H | H |

Erfindungsgemäße flüssigkristalline Mischungen enthalten neben ein oder mehreren Verbindungen der Formel I, zwei, drei oder mehr Komponenten ausgewählt aus den Tabellen A und B.

**Tabelle A: (L¹, L², L³ = H oder F)**

| | |
|---|---|
| 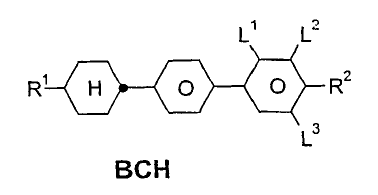 | 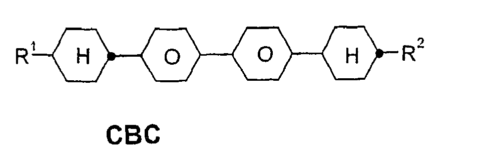 |
| 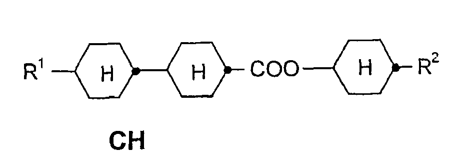 | 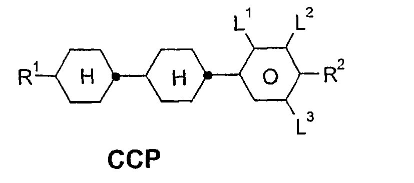 |
| 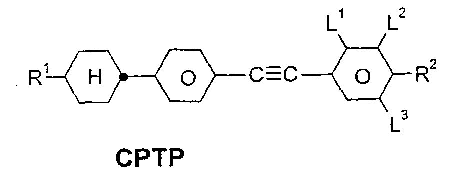 | 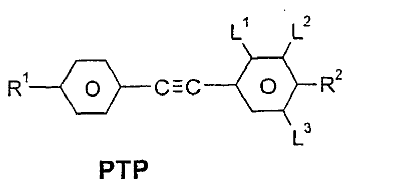 |
| 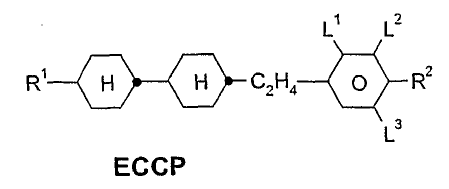 | 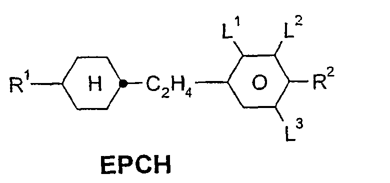 |
| 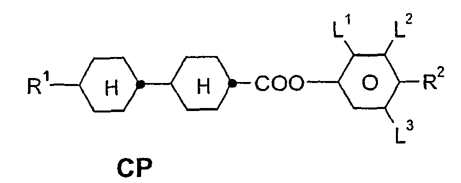 | 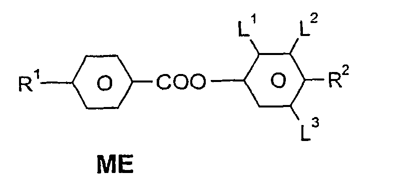 |
| 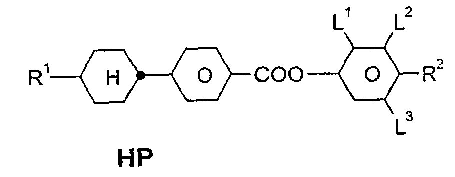 | 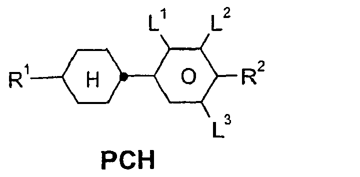 |
| 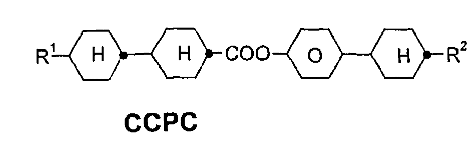 | 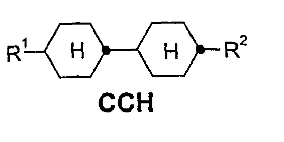 |
| 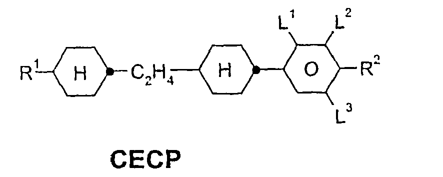 | |

**Tabelle B:**

| | |
|---|---|
| 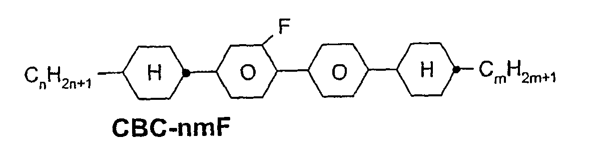 | |
| 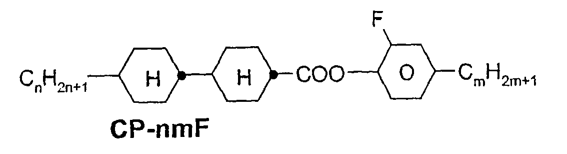 | |
| 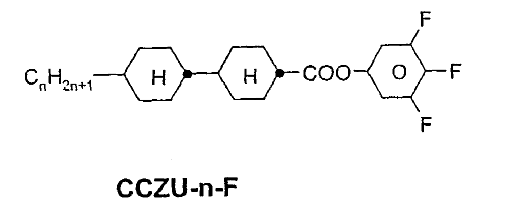 | |
| 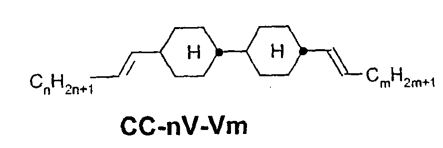 | 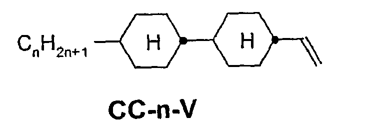 |
| 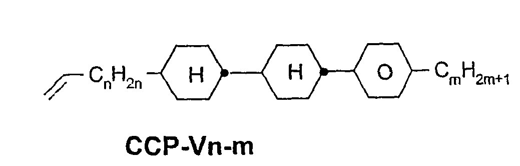 | 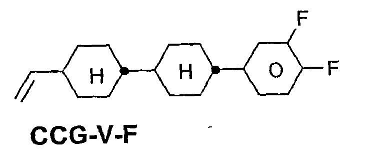 |
| 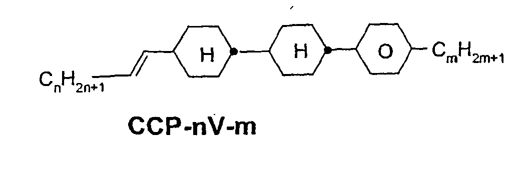 | 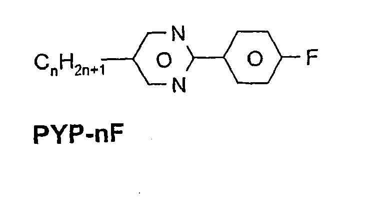 |
| 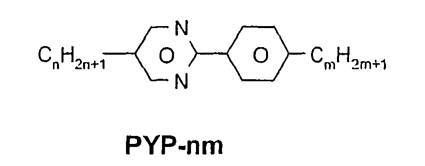 | 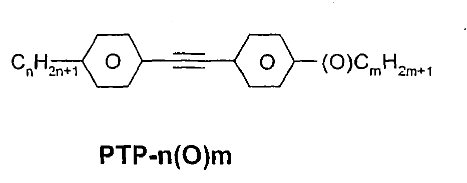 |
| 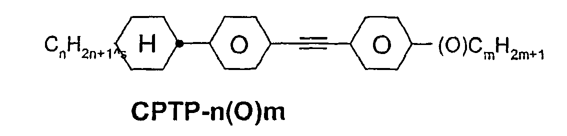 | |
| 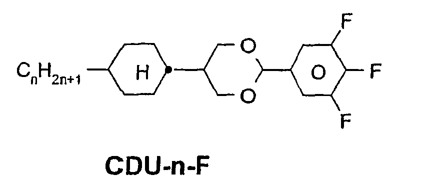 | |
| 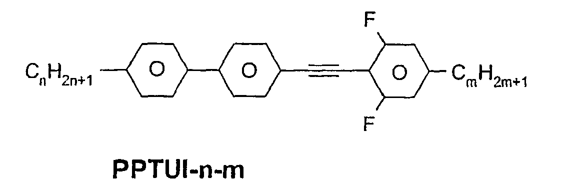 | |
| 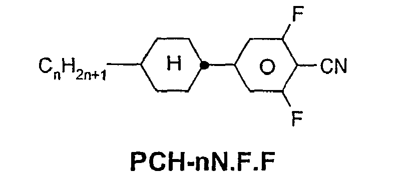 | 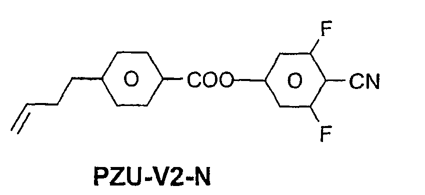 |
| 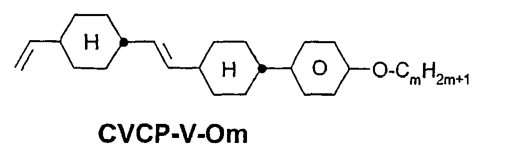 | |
| 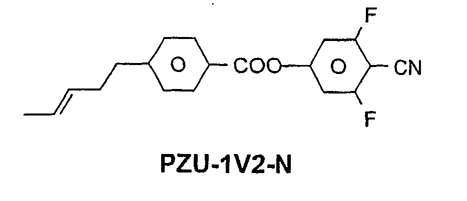 | |
| 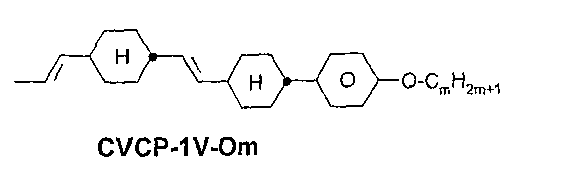 | |
| 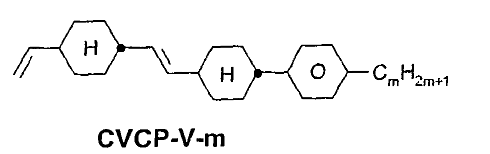 | |
| 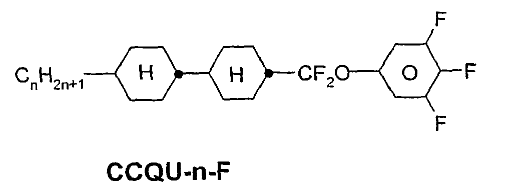 | |
| 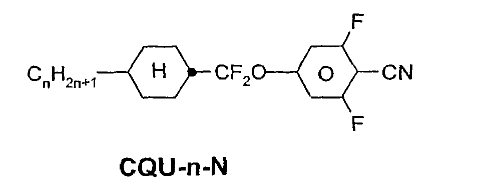 | |
| 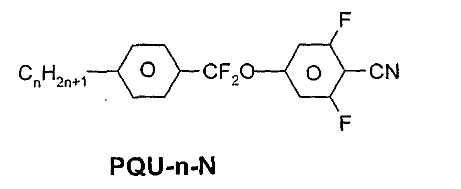 | |
| 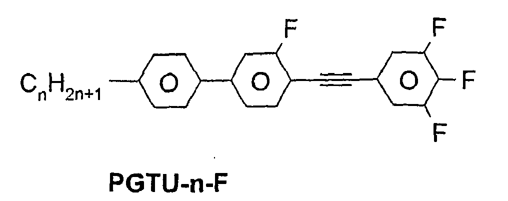 | |
| 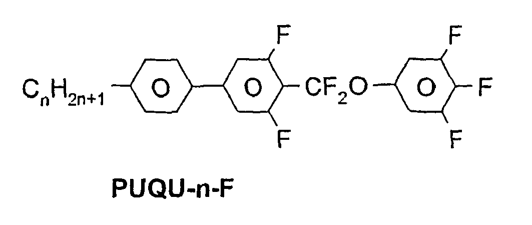 | |
| 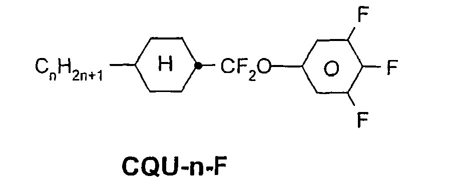 | |
| 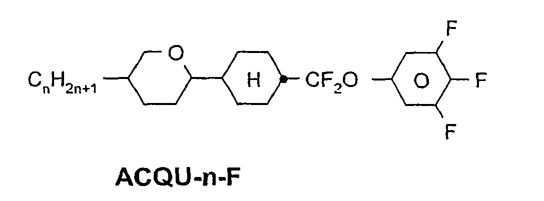 | |

### Tabelle C:

In der Tabelle C werden mögliche Dotierstoffe angegeben, die in der Regel den erfindungsgemäßen Mischungen zugesetzt werden.

| | |
|---|---|
| 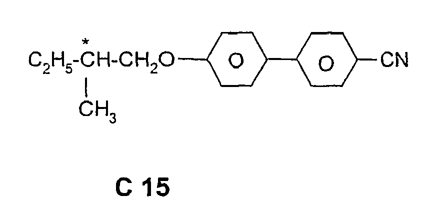 | 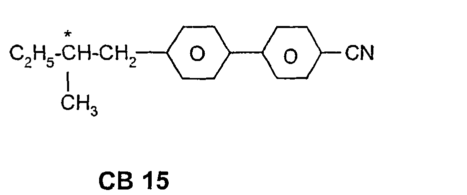 |
| 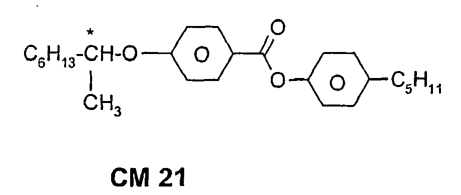 | |
| 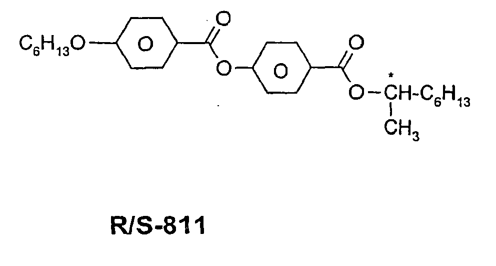 | |
| 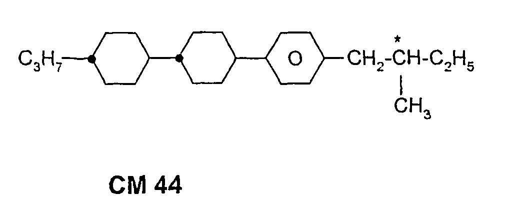 | |
| 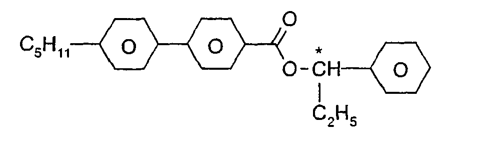 | |
| 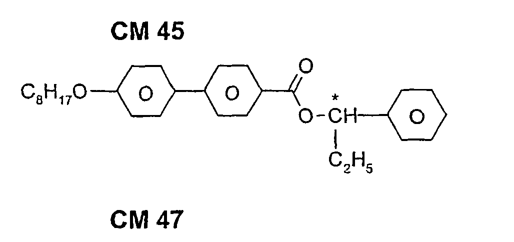 | |
| 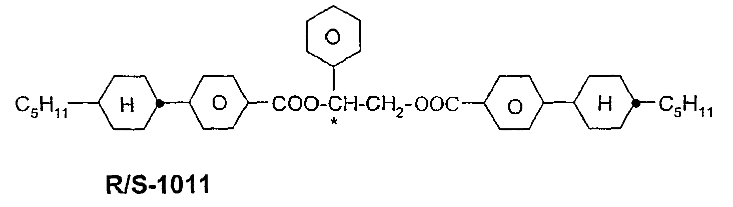 | |
| 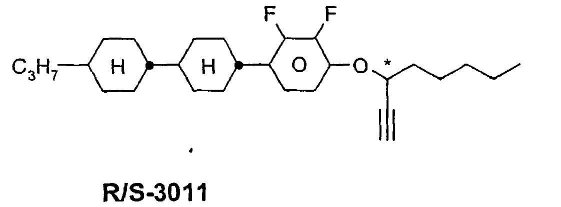 | |
| 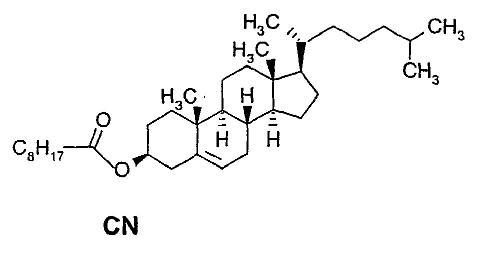 | |
| 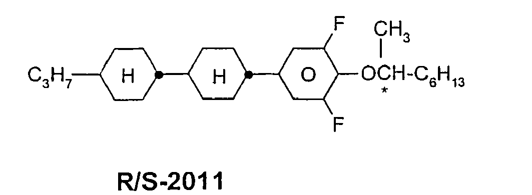 | |
| 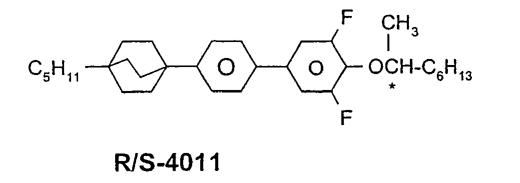 | |

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Fließviskosität ν₂₀ (mm²/sec) und die Rotationsviskosität γ₁ [mPa·s] wurden jeweils bei 20 °C bestimmt.

"Übliche Aufarbeitung" bedeutet: man gibt gegebenenfalls Wasser hinzu, extrahiert mit Dichlormethan, Diethylether, Methyl-tert.Butylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DAST: Diethylaminoschwefeltrifluorid
- DMEU: 1,3-Dimethyl-2-imidazolidinon
- KOT: Kalium-tertiär-butanolat
- THF: Tetrahydrofuran
- pTsOH: p-Toluolsulfonsäure

### Beispiel 1

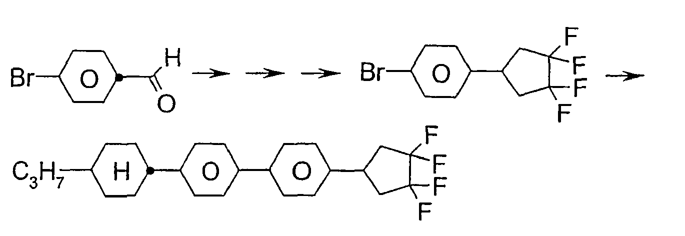

### Schritt 1.1

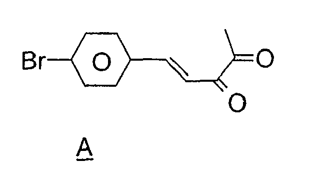

Eine Mischung aus 0,195 mol 4-Brombenzaldehyd und 0,185 mol 3,3-Dimethoxy-2-butanon in 200 ml Methanol wird mit 150 ml 20 %iger Natronlauge versetzt und 10 Stunden bei Raumtemperatur gerührt. Man destilliert das Methanol im Vakuum ab, extrahiert mit Methyl-tert.Butylether, trocknet über Natriumsulfat und dampft ein. Der Rückstand wird im Vakuum destilliert. Das Destillat wird mit 10 g p-Toluolsulfonsäure und 1 1 Aceton 15 Stunden bei Raumtemperatur gerührt, das Aceton abgedampft, der Rückstand in Methyl-tert.Butylether aufgenommen und mit Wasser neutral gewaschen. Man dampft ein und kristallisiert den Rückstand aus Pentan.

### Schritt 1.2

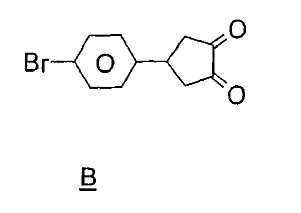

40 g Dion A werden in 400 ml Methanol gelöst und zu einer Lösung von 20 g Magnesiummethanolat in Methanol bei Siedehietze getropft. Man kocht 1 h, dampft das Methanol ab und säuert mit verd. Salzsäure an. Man extrahiert mit Methyl-tert.Butylether, trocknet und dampft ein. Der Rückstand wird durch Umkristallisieren aufgereinigt.

### Schritt 1.3

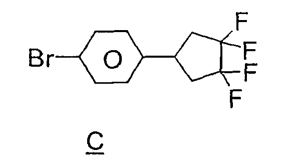

0,1 mol Bromphenylcyclopentadion B werden in einem Autoklaven mit 0,7 mol SF₄ bei 50 °C 12 h zur Reaktion gebracht. Nach dem Abkühlen auf Raumtemperatur und Ablasen der Reaktionsgase wird das Reaktionsgemisch in Dichlormethan aufgenommen, neutral gewaschen und das unpolare Produkt mittels Säulenchromatographie abgetrennt.

### Schritt 1.4

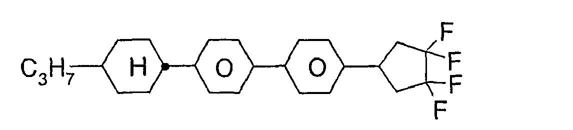

0,1 mol 4-Bromphenyltetrafluorcyclopentan C werden mit 0,1 mol 4-Propylcyclohexylphenylboronsäure in 200 ml Toluol gelöst und mit 100 ml 2 molarer Sodalösung und 0,002 mol Pd(PPh₃)₄-Katalysator versetzt. Man kocht 12 h am Rückfluß und arbeitet zuletzt wie üblich auf.

Analog werden die folgenden Verbindungen der Formel 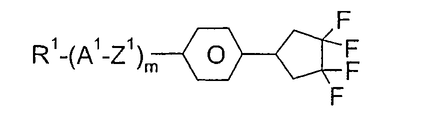 hergestellt:

| R¹ | -(A¹-Z¹)ₘ- |
|---|---|
| CH₃ | 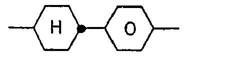 |
| C₂H₅ | 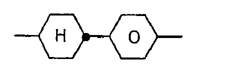 |
| n-C₄H₉ | 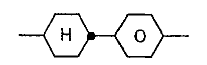 |
| n-C₅H₁₁ | 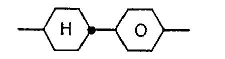 |
| n-C₆H₁₃ | 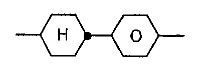 |
| n-C₇H₁₅ | 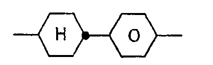 |
| CH₂=CH | 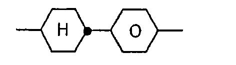 |
| CH₃CH=CH | 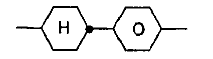 |
| CH₂=CHCH₂CH₂ | 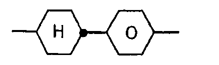 |
| CH₃CH=CHCH₂CH₂ | 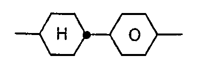 |
| CH₃ | 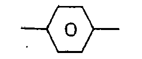 |
| C₂H₅ | 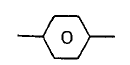 |
| n-C₃H₇ | 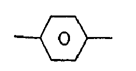 |
| n-C₄H₉ | 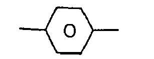 |
| n-C₅H₁₁ | 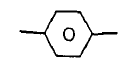 |
| n-C₆H₁₃ | 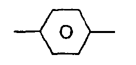 |
| n-C₇H₁₅ | 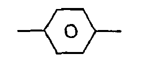 |
| CH₂=CH | 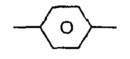 |
| CH₃-CH=CH₂ | 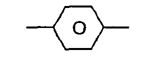 |
| CH₂=CHCH₂CH₂ | 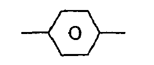 |
| CH₃CH=CHCH₂CH₂ | 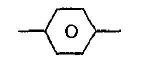 |
| CH₃ | 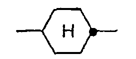 |
| C₂H₅ | 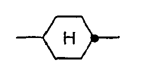 |
| n-C₃H₇ | 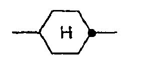 |
| n-C₅H₁₁ | 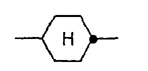 |
| CH₃ | 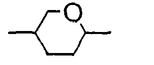 |
| C₂H₅ | 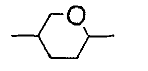 |
| n-C₃H₇ | 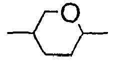 |
| n-C₅H₁₁ | 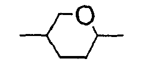 |

## Patentansprüche

1. 3,3,4,4-Tetrafluorcyclopentanverbindungen der Formel I, 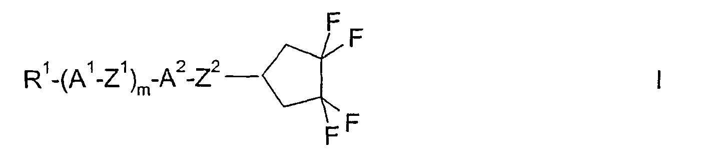 worin
R¹ H, einen unsubstituierten, einen einfach durch CN oder CF₃ oder einen mindestens einfach durch Halogen substituierten Alkylrest mit bis zu 15 C-Atomen, wobei in diesen Resten auch eine oder mehrere CH₂-Gruppen jeweils unabhängig voneinander durch -O-, -CH=CH-, 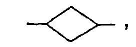 -CO-, -CO-O-, -O-CO- oder -O-CO-O- so ersetzt sein können, dass O-Atome nicht direkt miteinander verknüpft sind,
A¹ und A² jeweils unabhängig voneinander
(a) trans-1,4-Cyclohexylenrest, worin auch eine oder mehrere nicht benachbarte CH₂-Gruppen durch -O- und/oder -S-ersetzt sein können,
(b) 1,4-Phenylenrest, worin auch eine oder zwei CH-Gruppen durch N ersetzt sein können,
(c) 1,4-Cyclohexenylen,
(d) Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) bis (d) durch ein oder mehrere Fluoratome substituiert sein können,
Z¹ und Z² jeweils unabhängig voneinander -CO-O-, -O-CO-, -CH₂O-, -OCH₂-, -CH₂CH₂-, -CH=CH-, -C≡C-, -(CH₂)₄-, -CF₂O-, -OCF₂-, -C₂F₄-, -CH=CH-CH₂CH₂-, oder eine Einfachbindung, und
m 0, 1 oder 2
bedeuten.

2. Verbindungen der Formeln I1-I30 gemäß Anspruch 1: 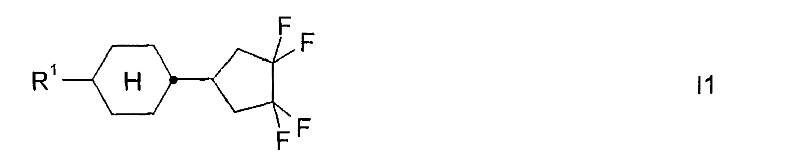 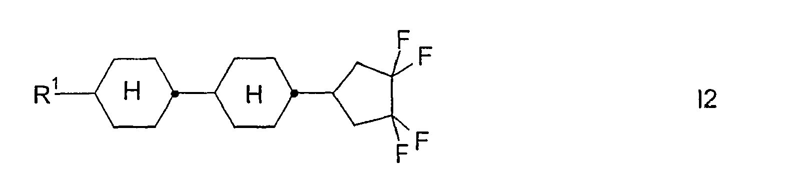 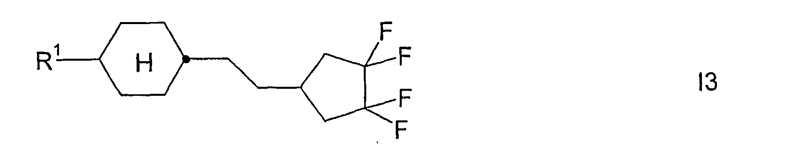 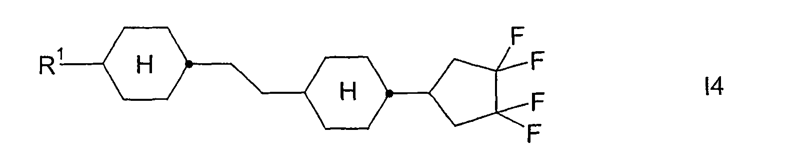 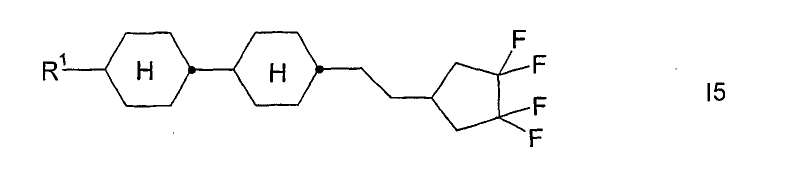 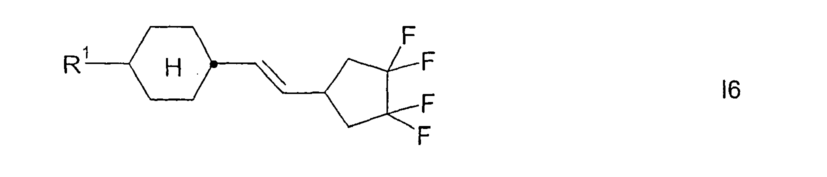 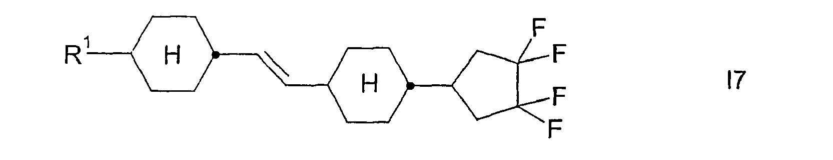 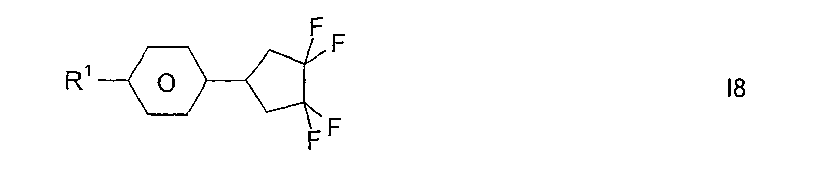 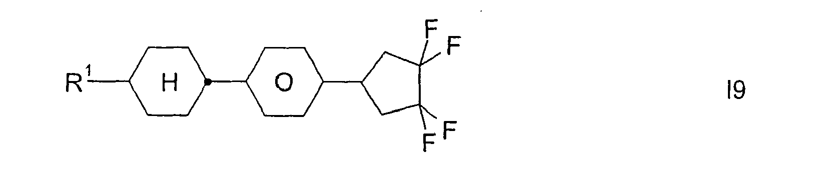 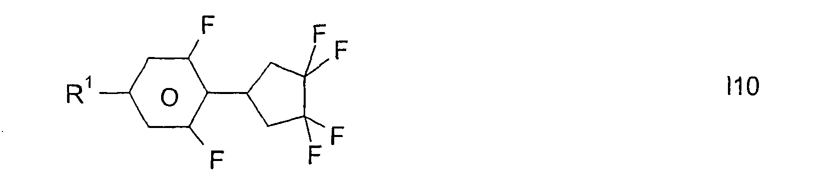 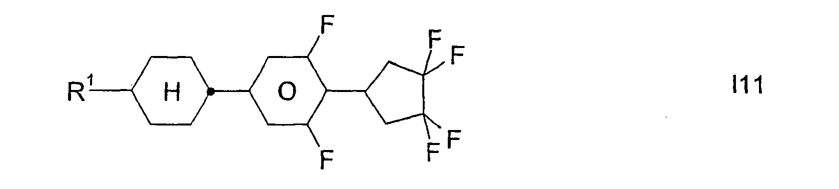 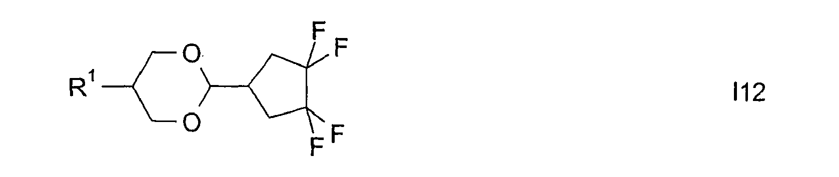 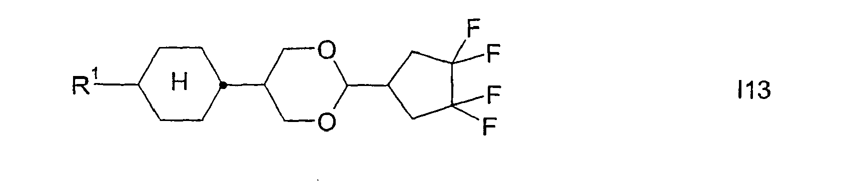 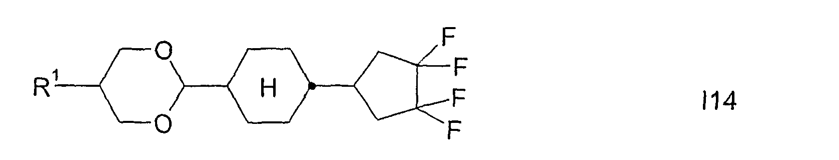 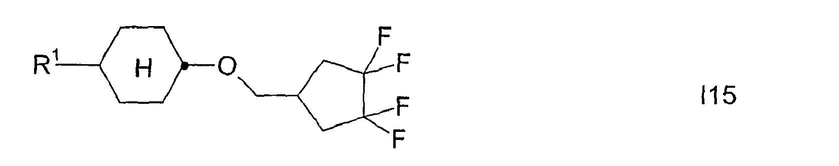 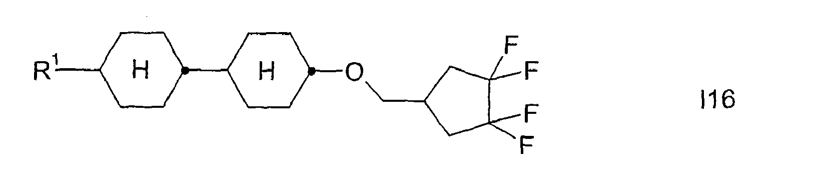 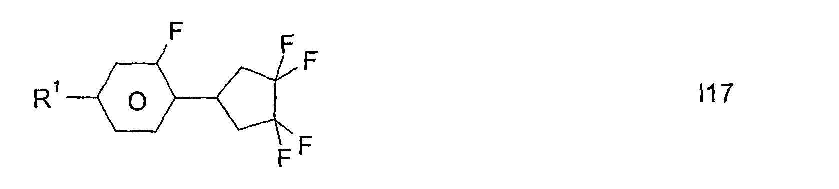 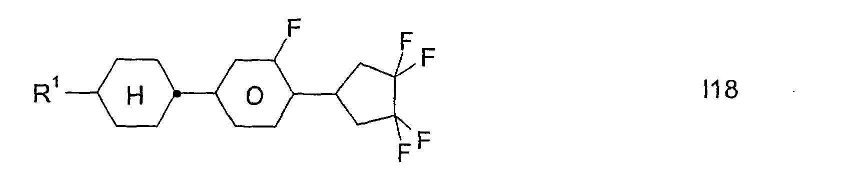 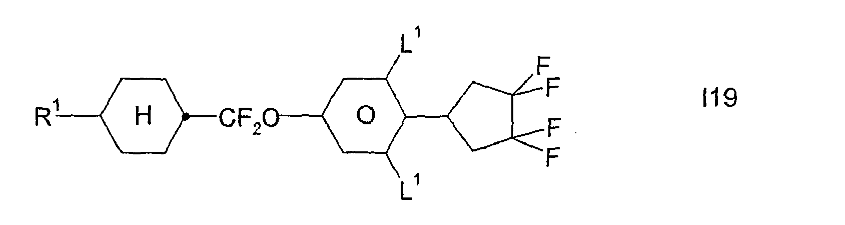 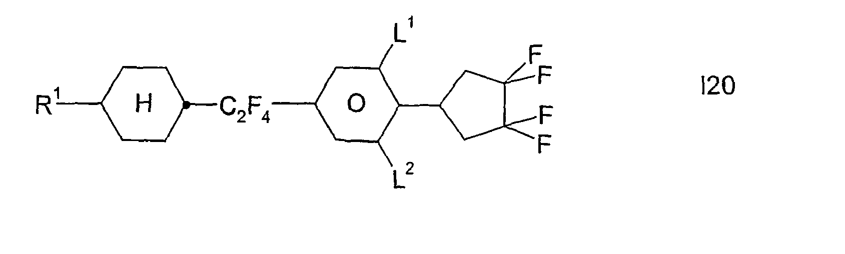 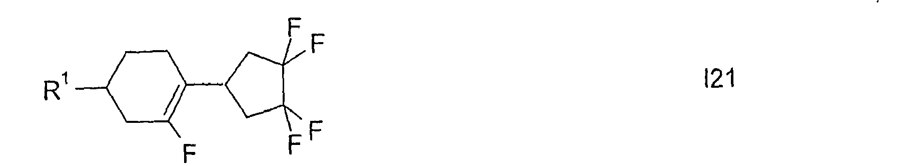 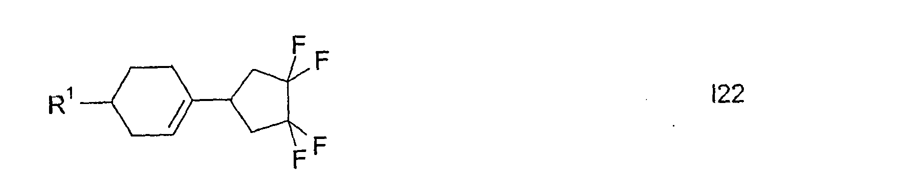 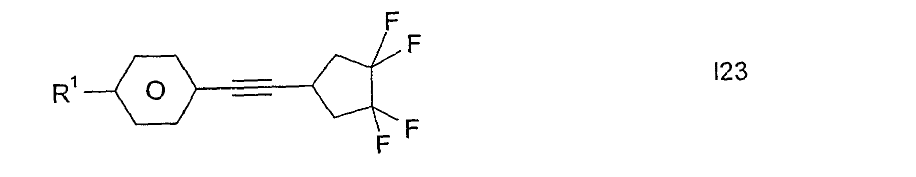 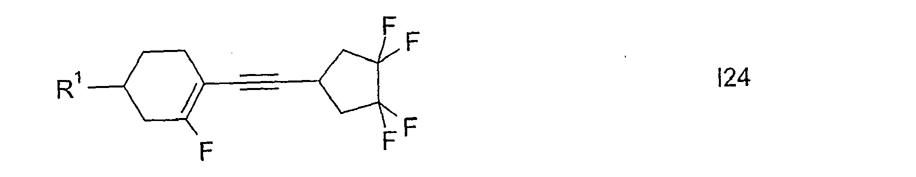 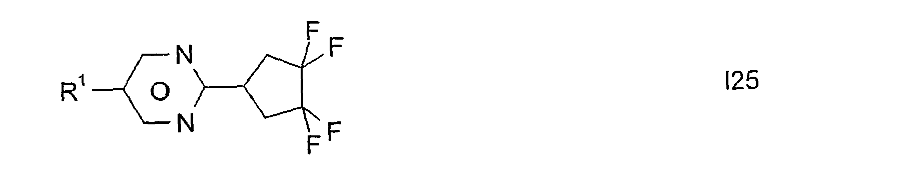 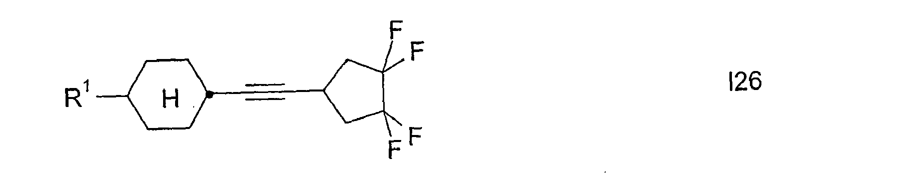 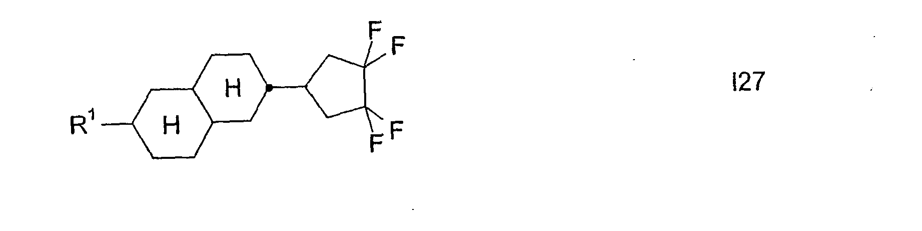 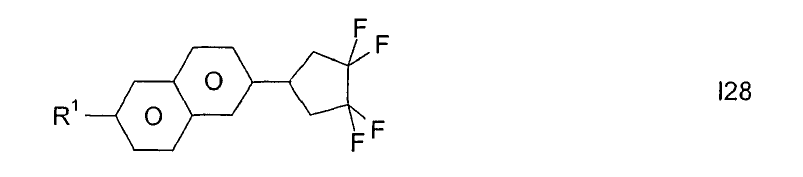 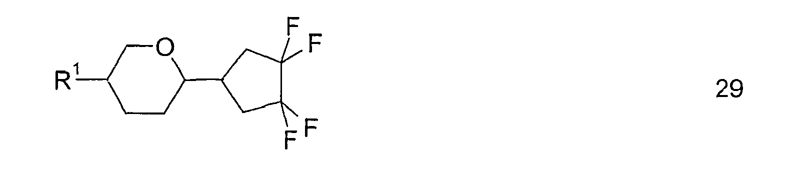 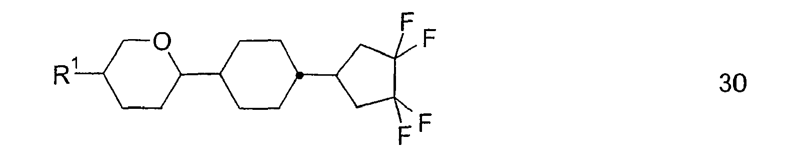 worin R¹ die in Anspruch 1 angegebene Bedeutung hat und L¹ und L² jeweils unabhängig voneinander H oder F bedeuten.

3. Verbindungen nach Anspruch 1 oder 2, **dadurch gekennzeichnet, dass** R¹ Alkyl, Alkoxy, Alkenyl oder Alkenyloxy mit bis zu 7 C-Atomen ist.

4. Verwendung von Verbindungen der Formel I als Komponenten flüssigkristalliner Medien.

5. Flüssigkristallines Medium mit mindestens zwei flüssigkristallinen Komponenten, **dadurch gekennzeichnet, dass** es mindestens eine Verbindung der Formel I enthält.

6. Flüssigkristall-Anzeigeelement, **dadurch gekennzeichnet, dass** es ein flüssigkristallines Medium nach Anspruch 5 enthält.

7. Elektrooptisches Anzeigeelement, **dadurch gekennzeichnet, dass** es als Dielektrikum ein flüssigkristallines Medium nach Anspruch 5 enthält.

## Claims

1. 3,3,4,4-Tetrafluorocyclopentane compounds of the formula I 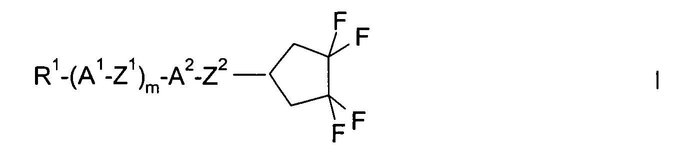 in which
R¹ denotes H, an alkyl radical having up to 15 C atoms which is unsubstituted, monosubstituted by CN or CF₃ or at least monosubstituted by halogen, where, in addition, one or more CH₂ groups in these radicals may each, independently of one another, be replaced by -O-, -CH=CH-, 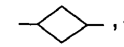 -CO-, -CO-O-, -O-CO- or -O-CO-O- in such a way that O atoms are not linked directly to one another,
A¹ and A² each, independently of one another, denote
(a) trans-1,4-cyclohexylene radical, in which, in addition, one or more non-adjacent CH₂ groups may be replaced by -O-and/or -S-,
(b) 1,4-phenylene radical, in which, in addition, one or two CH groups may be replaced by N,
(c) 1,4-cyclohexenylene,
(d) radical from the group 1,4-bicyclo[2.2.2]octylene, piperidine-1,4-diyl, naphthalene-2,6-diyl, decahydronaphthalene-2,6-diyl and 1,2,3,4-tetrahydronaphthalene-2,6-diyl,
where the radicals (a) to (d) may be substituted by one or more fluorine atoms,
Z¹ and Z² each, independently of one another, denote -CO-O-, -O-CO-, -CH₂O-, -OCH₂-, -CH₂CH₂-, -CH=CH-, -C≡C-, -(CH₂)₄-, -CF₂O-, -OCF₂-, -C₂F₄-, -CH=CH-CH₂CH₂- or a single bond, and
m denotes 0, 1 or 2.

2. Compounds of the formulae I1-I30 according to Claim 1: 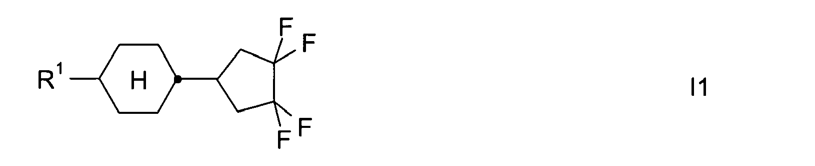 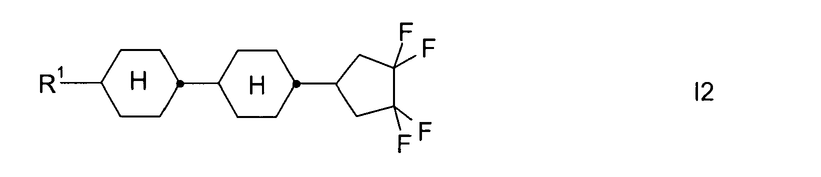 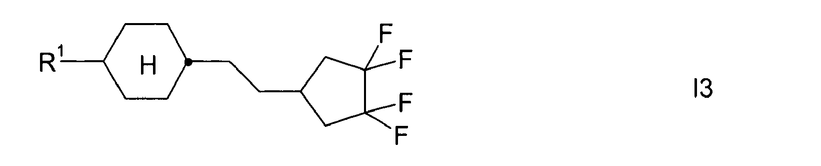 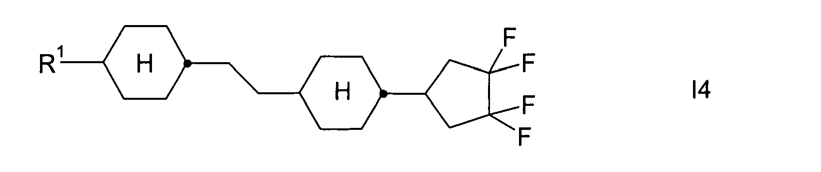 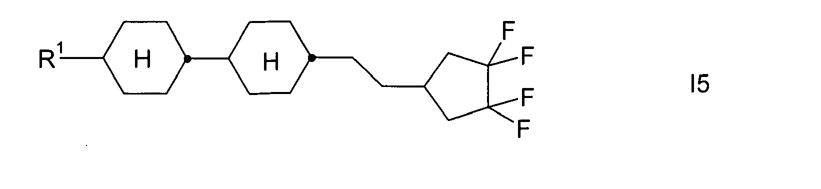 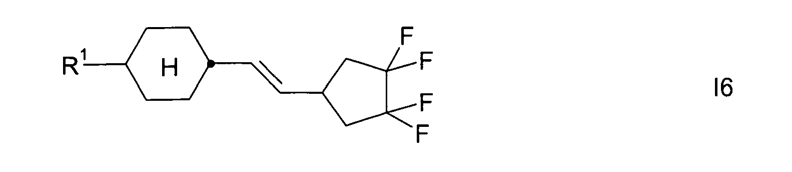 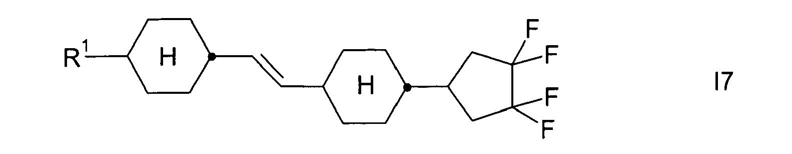 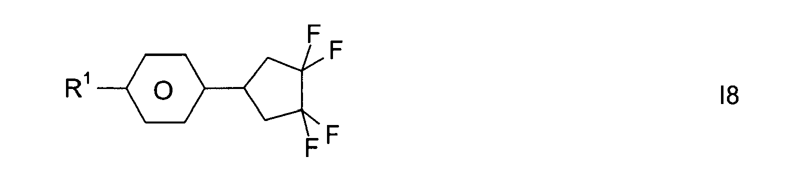 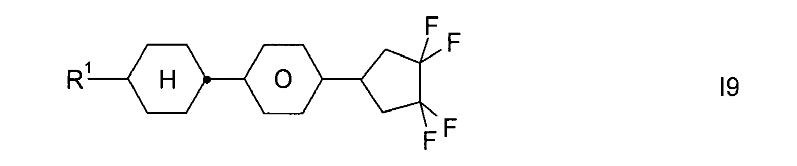 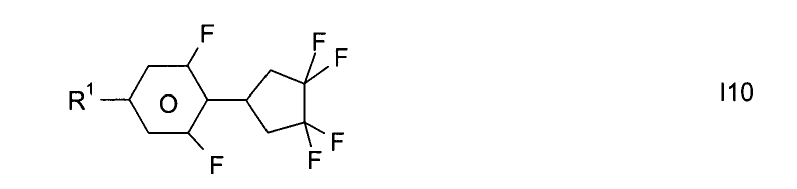 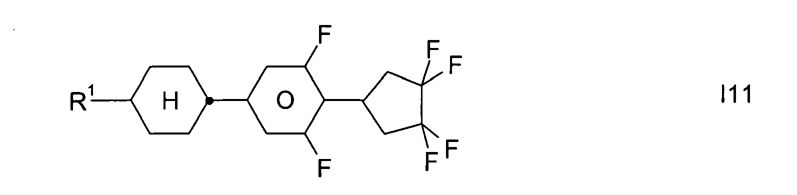 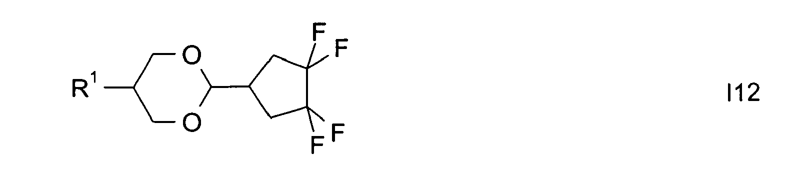 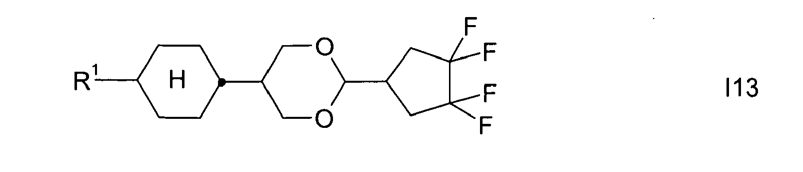 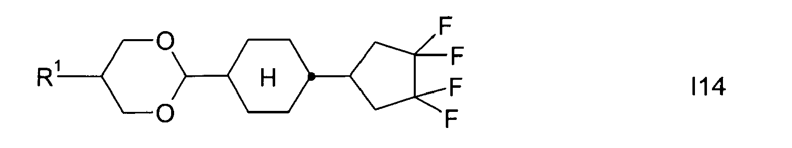 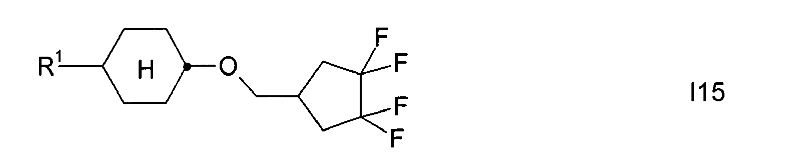 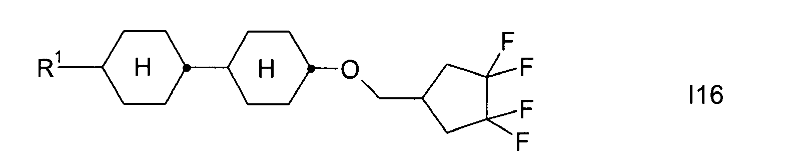 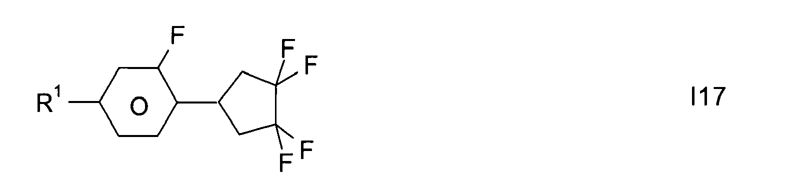 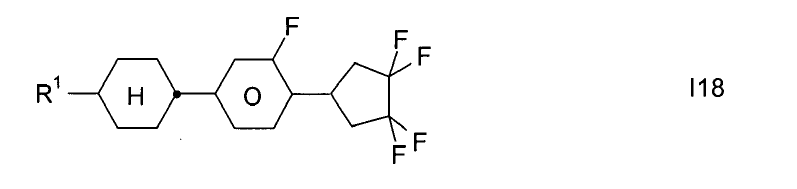 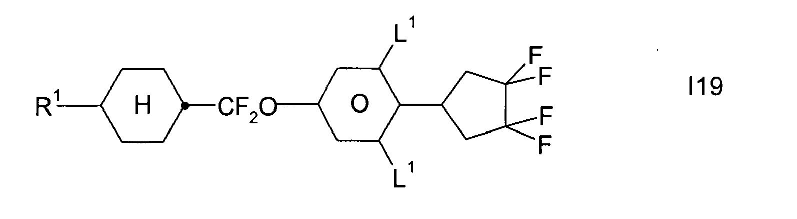 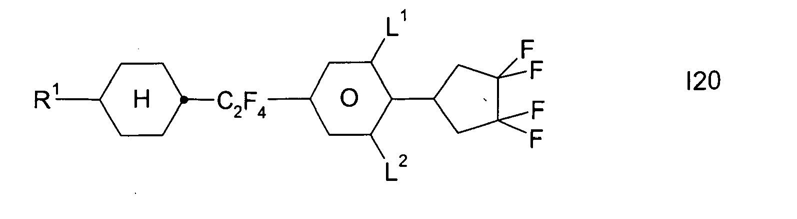 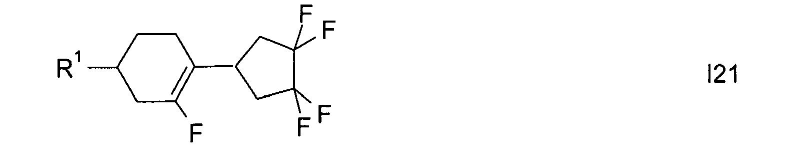 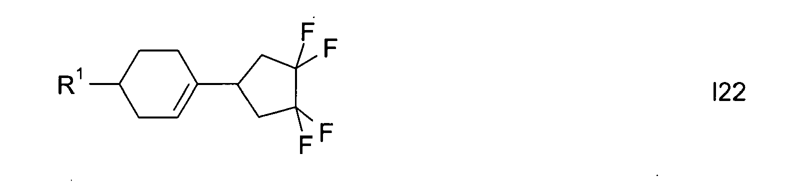 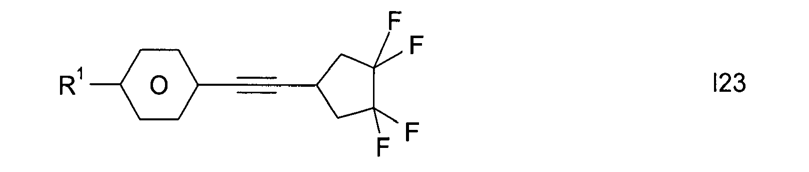 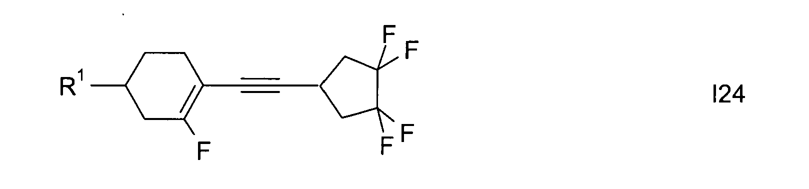 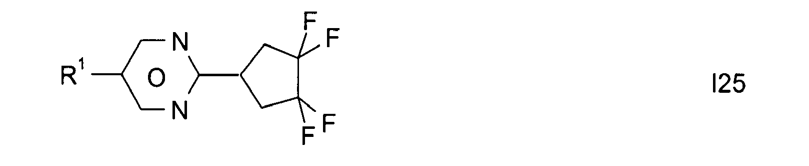 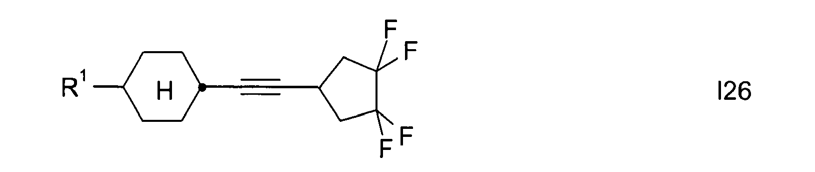 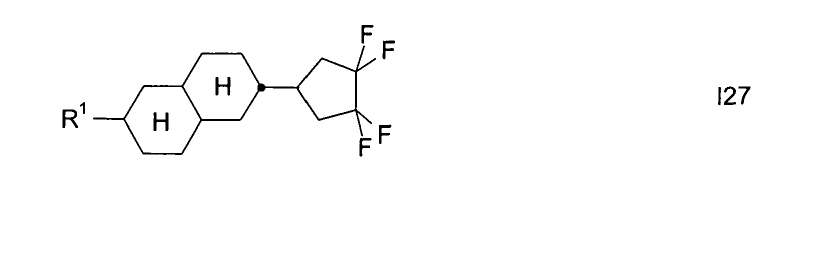 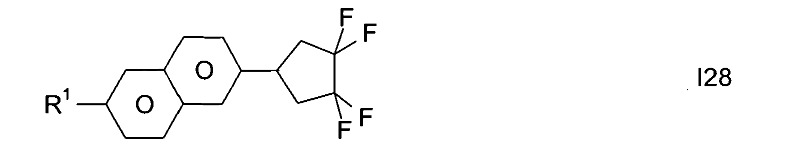 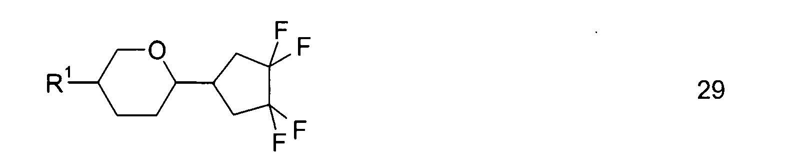 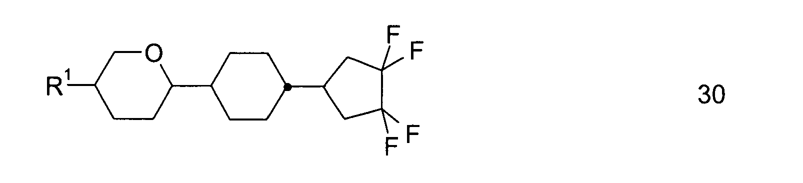 in which R¹ has the meaning indicated in Claim 1, and L¹ and L² each, independently of one another, denote H or F.

3. Compounds according to Claim 1 or 2, **characterised in that** R¹ is alkyl, alkoxy, alkenyl or alkenyloxy having up to 7 C atoms.

4. Use of compounds of the formula I as components of liquid-crystalline media.

5. Liquid-crystalline medium having at least two liquid-crystalline components, **characterised in that** it comprises at least one compound of the formula I.

6. Liquid-crystal display element, **characterised in that** it contains a liquid-crystalline medium according to Claim 5.

7. Electro-optical display element, **characterised in that** it contains, as dielectric, a liquid-crystalline medium according to Claim 5.

## Revendications

1. Composés de 3,3,4,4-tétrafluorocyclopentane de la formule I 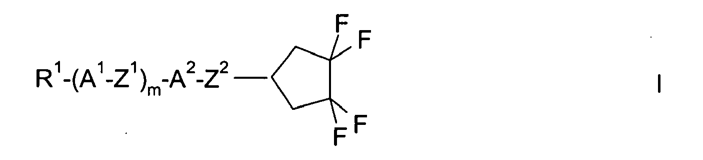 dans laquelle
R₁ représente H, un radical alkyle comportant jusqu'à 15 atomes de C qui est non substitué, monosubstitué par CN
ou CF₃ ou au moins monosubstitué par halogène, où, en plus, un ou plusieurs groupes CH₂ dans ces radicaux peuvent, chacun indépendamment les uns des autres, être remplacés par -O-, -CH=CH-, 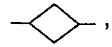 -CO-, -CO-O-, -O- CO- ou -O-CO-O- de telle sorte que des atomes de O ne soient pas liés directement les uns aux autres,
A¹ et A² chacun indépendamment de l'autre, représentent
(a) un radical trans-1,4-cyclohexylène, où, en plus, un ou plusieurs groupes CH₂ non adjacents peuvent être remplacés par -O- et/ou -S-,
(b) un radical 1,4-phénylène, où, en plus, un ou deux groupes CH peuvent être remplacés par N,
(c) 1,4-cyclohexénylène,
(d) un radical pris parmi le groupe 1,4-bicyclo[2.2.2]octylène, pipéridine-1,4-diyle, naphthalène-2,6-diyle, décahydronaphthalène-2,6-diyle et 1,2,3,4-tétrahydronaphthalène-2,6-diyle,
où les radicaux (a) à (d) peuvent être substitués par un ou plusieurs atomes de fluor,
Z¹ et Z² chacun indépendamment de l'autre, représentent -CO-O-, -O-CO-, -CH₂O-, -OCH₂-, -CH₂CH₂-, -CH=CH-, -C≡C-, -(CH₂)₄-, -CF₂O- -OCF₂-, -C₂F₄-, -CH=CH-CH₂CH₂- ou une liaison simple, et
m représente 0, 1 ou 2.

2. Composés des formules 11-130 selon la revendication 1: 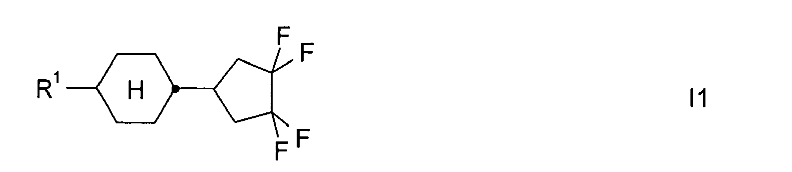 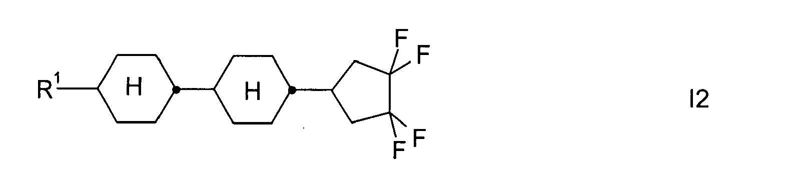 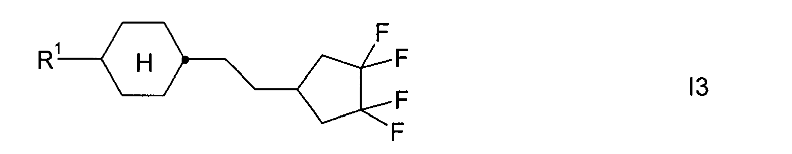 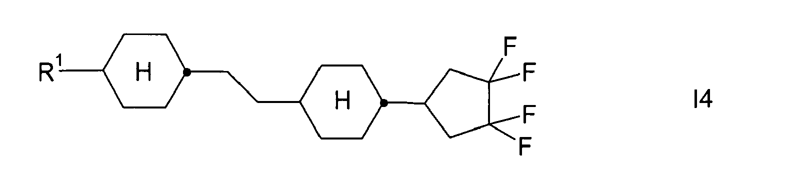 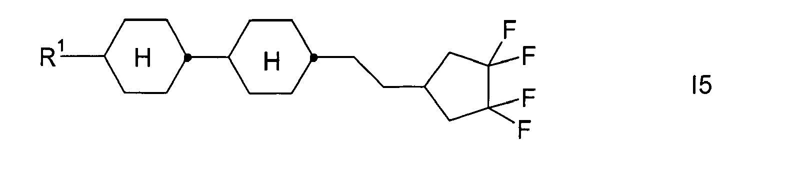 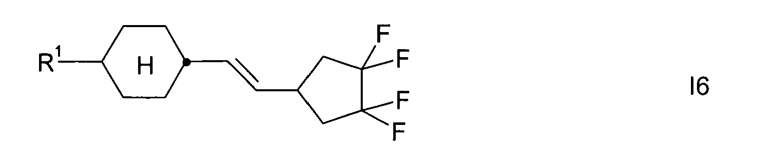 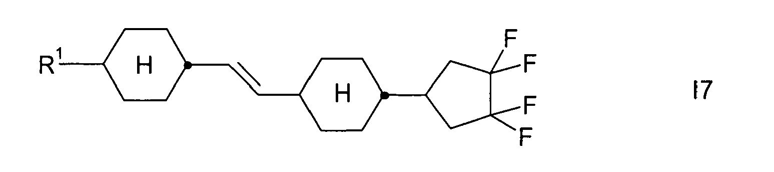 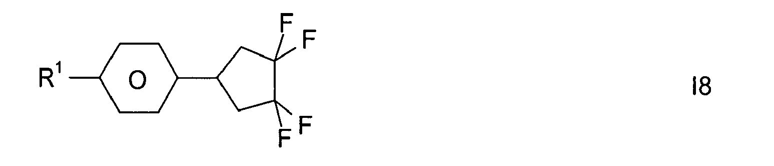 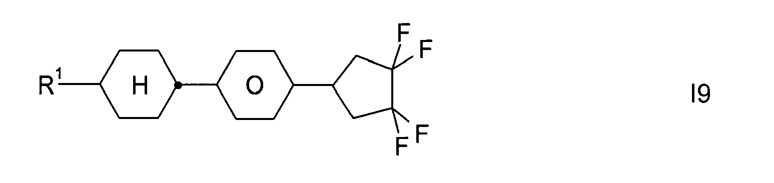 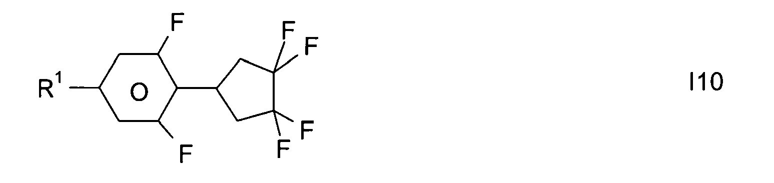 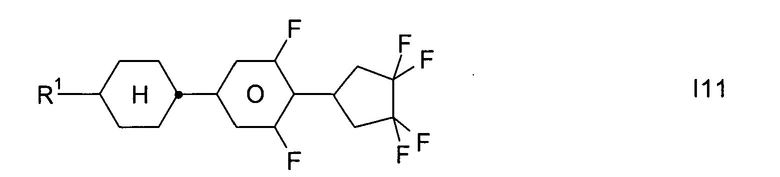 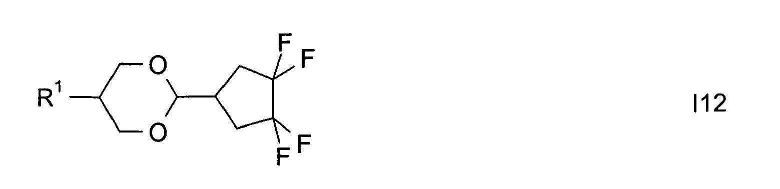 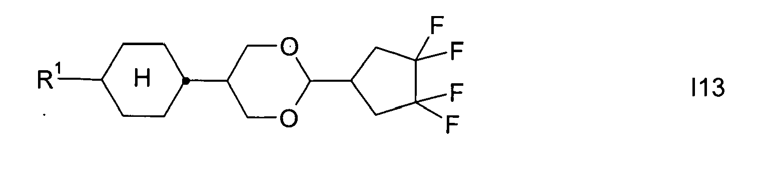 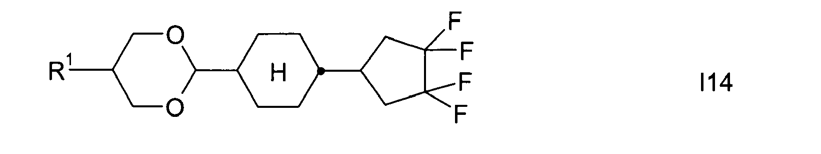 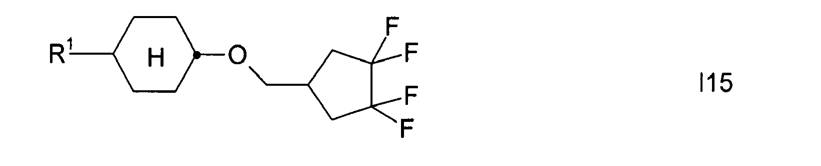 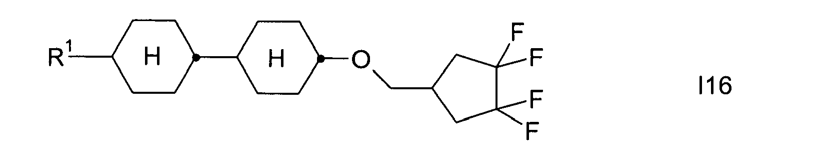 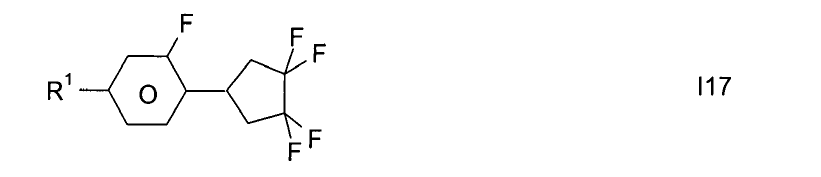 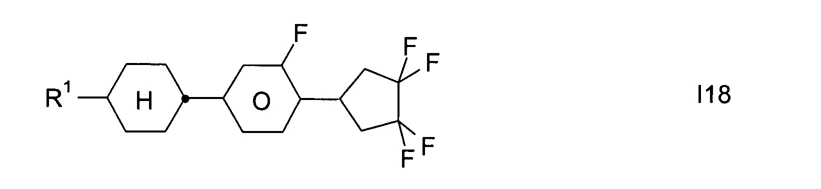 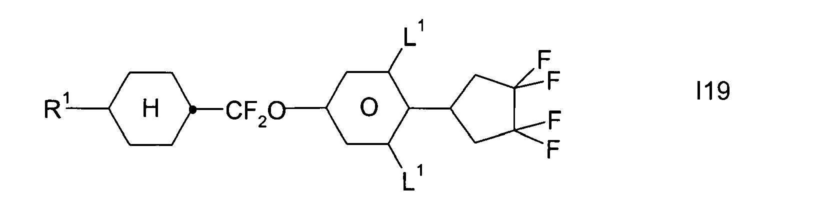 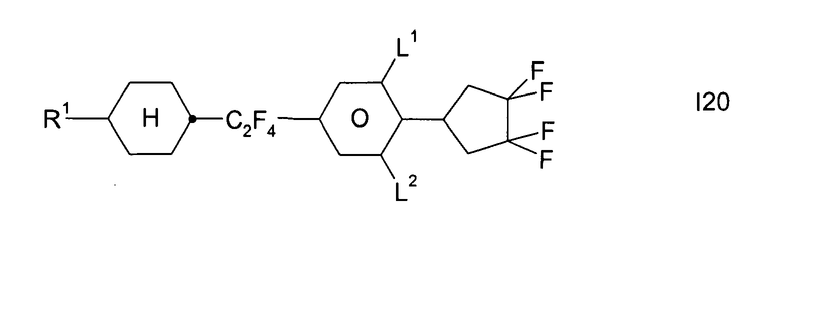 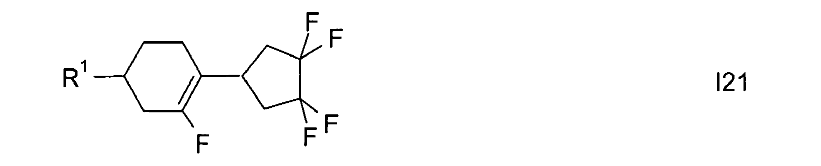 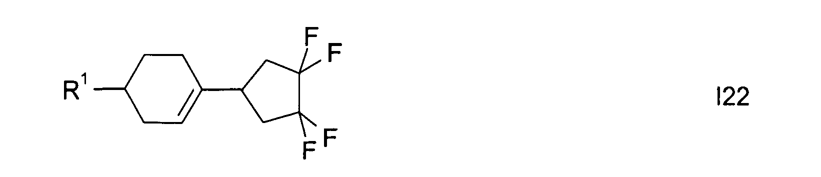 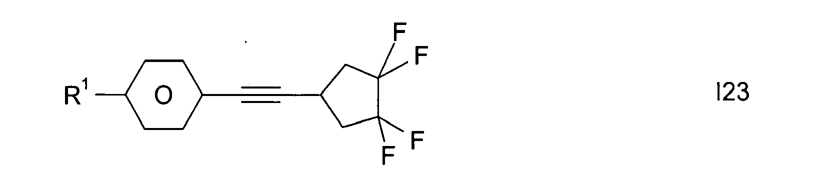 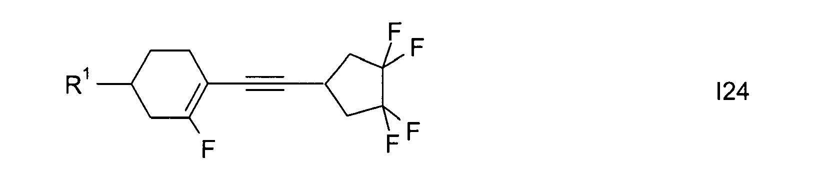 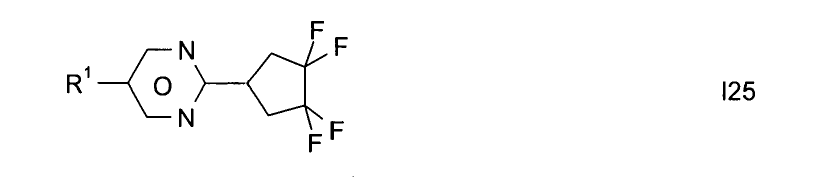 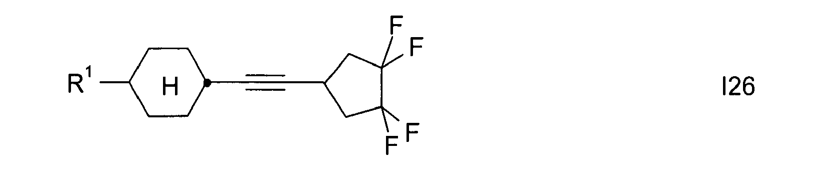 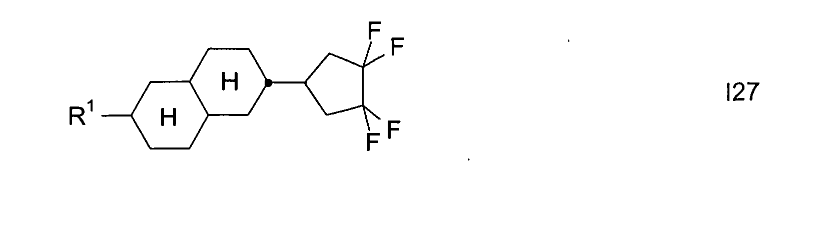 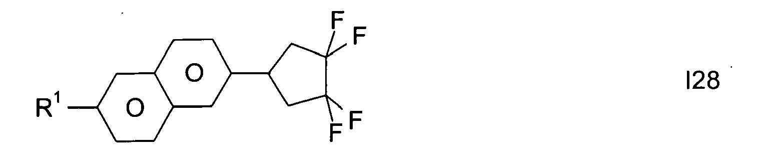 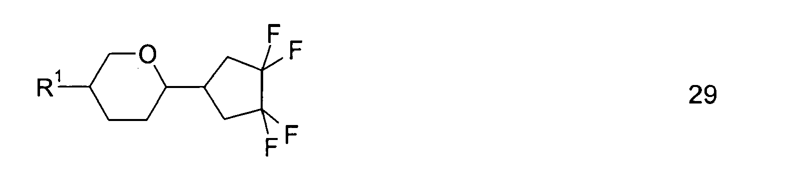 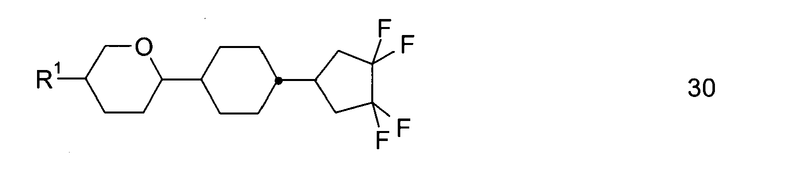 dans lesquelles R¹ présente la signification indiquée dans la revendication 1, et L¹ et L², chacun indépendamment de l'autre, représentent H ou F.

3. Composés selon la revendication 1 ou 2, **caractérisés en ce que** R¹ est alkyle, alkoxy, alkényle ou alkényloxy comportant jusqu'à 7 atomes de C.

4. Utilisation de composés de la formule I en tant que composants de milieux de cristal liquide.

5. Milieu de cristal liquide comportant au moins deux composants de cristal liquide, **caractérisé en ce qu'**il comprend au moins un composé de la formule I.

6. Elément d'affichage à cristaux liquides, **caractérisé en ce qu'**il contient un milieu de cristal liquide selon la revendication 5.

7. Elément d'affichage électro-optique, **caractérisé en ce qu'**il contient, en tant que diélectrique, un milieu de cristal liquide selon la revendication 5.
